# EUROPEAN PATENT APPLICATION

(11) **EP 2 239 577 A1**
(43) Date of publication of application: **13.10.2010**
(21) Application number: 09157464.0
(22) Date of filing: 07.04.2009
(51) Int. Cl.: G01N 33/574, C07K 14/00, A61K 49/00

(54) **Protein-based methods and compositions for the diagnosis of colorectal adenocarcinoma**

(71) Applicant: Vereniging voor Christelijk Hoger Onderwijs, Wetenschappelijk Onderzoek en Patiëntenzorg, 1081 HV Amsterdam (NL)
(72) Inventor: The designation of the inventor has not yet been filed
(74) Representative: Jungen, Carmen

(57) **Abstract**

Protein-based methods and compositions for the diagnosis of colorectal adenocarcinoma are disclosed. A method for indentifying cell-surface proteins, which are transmembrane proteins or proteins with a signal peptide and which are over-expressed in colorectal cancer (CRC) is disclosed. Biomarkers found with this method, diagnostic methods using them and contrast agents directed to them for use in magnetic resonance imaging (MRI) and/or magnetic photon imaging (MPI) are disclosed.
The methods and biomarkers allow for differentiating progressive (high-risk) CRC (adenocarcinomas) from non-progressive (low-risk) colorectal adenomas.

## Description

### SUBJECT OF THE INVENTION

The present invention relates to contrast agents, diagnostic markers and methods for detecting colorectal adenocarcinoma.

### BACKGROUND OF THE INVENTION

Most cancers are epithelial in origin and arise through stepwise progression from normal cells, through dysplasia, into malignant cells that invade surrounding tissues and have metastatic potential. Colorectal cancer (CRC; also referred to as colon cancer or large bowl cancer) is one prominent type of cancer undergoing such tumour progression.

CRC includes cancerous growth in the colon, rectum and appendix. It is one of the most significant human cancers with an incidence of about 1,000,000 new cases worldwide every year. Thus, CRC is the third most common cancer and the fourth leading cause of cancer-related deaths in the world (the second leading cause in the Western world; reviewed, e.g. in Gryfe, R. et al. (1997) Curr. Probl. Cancer 21, 233-300; Petersen, G. M. et al (1999) Cancer 86, 2540-2550.

CRC is curable if diagnosed at an early stage (i.e. stage I) of tumour development. The five-year survival rate for early stage CRC is >90%. At this early stage, most patients have no phenotypic symptoms of the disease. Early detection can markedly improve chances of long-term survival, as the five-year survival rate for CRC at a late stage (i.e. stage IV) of tumour development is only <5%. More than 95% of the cases of CRC are manifested as adenocarcinomas (Muto, T. et al. (1975) Cancer 36, 2251-2270; Fearon, E. R. et al. (1990) Cell 61, 759 767).

In the past, screenings have been undertaken to identify genes which are implicated in the progression from low-risk colorectal adenomas to high-risk adenocarcinomas. To this end, genes were identified, the expression of which is either up- or down-regulated in colorectal adenocarcinomas versus adenomas (see e.g. Carvalho et al. (2009) Gut 58, 79-89).

Currently, colonoscopy is the standard screening modality for CRC having the highest sensitivity and specificity to detect colorectal tumours among techniques that are currently applied.

Nevertheless, screening by colonoscopy may have disadvantages. Among those are (i) that colonoscopy is an invasive technique being a limiting factor when CRC screening is offered for asymptomatic individuals, (ii) that there is a small but significant risk of bowel perforation as a consequence of colonoscopy and (iii) that colonoscopy cannot discriminate between non-progressive (low-risk) and progressive (high-risk) colon tumour lesions.

Thus, there is a continuing need for agents, compositions, markers and methods that allow diagnosis of CRC.

### OBJECTIVE AND SUMMARY OF THE INVENTION

It is one objective of the present invention to provide for methods that allow diagnosing CRC. It is a further objective of the present invention to provide for markers that can be used for diagnosing CRC.

Yet another objective of the present invention is concerned with the provision of contrast agents which can be used in the detection of CRC.

These and other objectives as they will become apparent from the ensuing description hereinafter form the subject matter of the independent claims. Some of the preferred embodiments of the present invention form the subject matter of the dependent claims.

The present invention in one embodiment thus relates to a diagnostic marker, preferably for detecting CRC comprising at least one polypeptide of the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31; and/or
- A polypeptide of SEQ ID No.: 32.

The present invention in the further embodiments relates to the use of at least one polypeptide selected from the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31, and/or
- A polypeptide of SEQ ID No.: 32.
as a diagnostic marker, preferably for detecting CRC.

In one embodiment, such use is performed outside the human or animal body.

Yet another aspect of the present invention relates to a contrast agent, optionally for use in magnetic resonance imaging (MRI) and/or magnetic photon imaging (MPI) comprising at least at least one compound being capable of interacting with a polypeptide selected from the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31; and/orA polypeptide of SEQ ID No.: 32.

In a preferred embodiment such compounds are coupled to marker molecules which preferably are detectable by MRI or MPI.

The compounds may preferably be antibodies.

Such contrast agents may preferably be used for detecting CRC.

In a further embodiment the present invention relates to use of at least one antibody capable of interacting with a polypeptide selected from the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31; and/or
- A polypeptide of SEQ ID No.: 32
as a contrast agent, optionally suitable for MRI and/or MPI.

Such contrast agents may preferably be used for detecting CRC.

Another embodiment of the present invention relates to a method of diagnosing CRC comprising at least the following steps:
a) Obtaining at least one sample from at least one human or animal individual suspected to suffer from ongoing or imminent CRC development;
b) Testing in said at least one sample for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
c) Testing in at least one control sample obtained from at least one human or animal individual not suffering from ongoing or imminent CRC development for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
d) Determining difference in expression of steps b) and d);
e) Deciding on the presence or imminence of colorectal cancer development based on the results obtained in step d).

In one embodiment, steps b), c), d) and/or e) of this method of diagnosis are performed outside the human or animal body.

Yet another aspect of the present invention relates to a method of diagnosing colorectal cancer comprising at least the following steps:
a) Testing in at least one human or animal individual suspected to suffer from ongoing or imminent CRC development for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
b) Comparing expression as determined in step a) with expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
   as determined for human or animal individuals not suffering from ongoing or imminent CRC development
c) Deciding on the presence or imminence of CRC development based on the results obtained in step b).

Another aspect of the present invention relates to a method of data acquisition comprising at least the following steps:
a) Testing in at least one human or animal individual suspected to suffer from ongoing or imminent CRC development for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
b) Comparing expression as determined in step a) with expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
as determined for human or animal individuals not suffering from ongoing or imminent CRC development.

Further, an embodiment of the present invention relates to a method of identifying at least one target molecule suitable as diagnostic marker for colorectal cancer, wherein the method comprises at least the following steps:
a) Obtaining cells from different human individuals all suffering from colorectal cancer and/or obtaining colorectal cancer cell lines;
b) Labeling polypeptides on the surface of said cells;
c) Isolating labelled polypeptides from non-labelled polypeptides;
d) Identification of isolated labelled polypeptides from the cell surface polypeptide-fraction of step c),
e) Of the polypeptides of step d), selecting those isolated labelled polypeptides for which genomic expression data indicate an increased expression compared to cells obtained from healthy individuals;
f) Of the polypeptides selected in step e), further selecting those isolated labelled polypeptides which comprise at least one transmembrane domain and/or at least one signal peptide;
g) Of the polypeptides selected in step f), further selecting those polypeptides with a positive RSC;
h) Of the polypeptides selected in step g), further selecting those polypeptides found within at least 70% of tested cells from different human individuals;
i) Of the polypeptides selected in step h), further selecting those polypeptides for which histological analysis confirms localization to the plasma membrane.

The aforementioned diagnostic markers, contrast agents, uses and methods are suitable for differentiating progressive (high-risk) CRC (adenocarcinomas) from non-progressive (low-risk) colorectal adenomas.

Further, the diagnostic markers, contrast agents, uses and methods of the present invention may be particularly suitable for non-invasive molecular imaging, for instance by MRI, for diagnosis of CRC.

Other embodiments of the present invention will become apparent from the detailed description hereinafter.

### FIGURE LEGENDS

Fig. 1 depicts SDS-PAGE analysis ofNeuravidine-purified biotin-labelled cell surface or intracellular fractions of colorectal cancer cell lines Colo 205, HT-29, Caco2, RKO and HCT116. The two lanes of intracellular fractions correspond to the fractions obtained from the CRC cell lines Colo 205, HT29 and Caco2 (left lane) and RKO and HCT116 (right lane).
Fig. 2 depicts histological analysis of PRNP (SEQ ID NO: 6).
Fig. 3 depicts histological detection of BCAM (SEQ ID NO: 5) in tissue culture obtained from colorectal cancer patients.

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated. In the context of the present invention, the terms "about" or "approximately" denote an interval of accuracy that the person skilled in the art will understand to still ensure the technical effect of the feature in question. The term typically indicates deviation from the indicated numerical value of ±10%, and preferably of ±5%.

Further definitions of terms will be given in the following in the context of which the terms are used.

As mentioned in the background section, identification of molecular marker (patterns) for diagnosing CRC has focussed on the identification of genes the expression of which is either up- or down-regulated during CRC development. While such data provides valuable information, the diagnostic markers, i.e. genes and their products (e.g. polypeptides) may not all be suitable for non-invasive molecular imaging diagnostic approaches.

The inventors of the present invention have succeeded in identifying a set of polypeptides which are likely to be over-expressed in the majority of patients suffering from colorectal cancer development versus healthy individuals and which may be accessible for non-invasive molecular imaging diagnostic methods.

To this end, the inventors have devised a screening strategy comprising at least the following steps:
a) Obtaining cells from different human individuals all suffering from colorectal cancer and/or obtaining colorectal cancer cell lines;
b) Labeling polypeptides on the surface of said cells;
c) Isolating labelled polypeptides from non-labelled polypeptides;
d) Identification of isolated labelled polypeptides from the cell surface polypeptide-fraction of step c),
e) Of the polypeptides of step d), selecting those isolated labelled polypeptides for which genomic expression data indicate an increased expression compared to cells obtained from healthy individuals;
f) Of the polypeptides selected in step e), further selecting those isolated labelled polypeptides which comprise at least one transmembrane domain and/or at least one signal peptide;
g) Of the polypeptides selected in step f), further selecting those polypeptides with a positive RSC;
h) Of the polypeptides selected in step g), further selecting those polypeptides found within at least 70% of tested cells from different human individuals;
i) Of the polypeptides selected in step h), further selecting those polypeptides for which histological analysis confirms localization to the plasma membrane.

The cells of step a) which are obtained from human individuals suffering from CRC will typically be cell types that are known to be involved in the development of CRC. Typically, the cells will comprise embryonic, fetal or adult stem cells; progenitor cells; blood cells such as B and T-lymphocytes, monocytes and macrophages; epithelial cells; fibroblasts; and neuronal cells. Epithelial cells may be preferred.

The cells used in step a) may also be established CRC cell lines. Such cell lines are commercially available and include *inter alia* Colo205, HT-29, Caco-2, RKO, HCT116, SW1398, LS513, SW480, SW620 and COLO 320..

The labellng step b) is undertaken to allow for efficient purification, enrichment and isolation of preferentially polypeptides that localise to the surface of cells. Thus, one will use labels which allow for efficient purification, e.g. by affinity chromatography. Such labels may include e.g. biotin, maltose binding protein (MBP), Glutathione-S-transferase (GST), histidine-tags, Flag-tags, antibodies and the like.

The labels may be covalently or non-covalently attached to proteins being preferentially located on the cell surface.

For covalent modification one may use coupling chemistries that are commonly used for such purposes. Thus, one may use homo and/or hetero-bi and/or multifunctional crosslinking agents. Typical cross-linking agents include but are not limited to bis (sulfosuccinimid) bis (diazo-benzidine), Dimethyl Adipimidate, Dimethyl Pimelimidate, Dimethyl Suberaimidate, Disuccininnclyl Suberate, Glutaraldehyde, in-Maleimidobenzoyl-N-Hydroxysuccinimide, Sulfosuccinidyl 4-(N-Maleimidomethyl) Cyclohexane-1-carboxylate etc.

A preferred label may be sulfo-NHS-SS-biotin.

Subsequently, the cells are typically lysed. This may be achieved by hypotonic, enzymatic, ultrasound or mechanical cell rupture.

Subsequently, labelled polypeptides may be isolated making use of the label. The person skilled in the art knows how to select the appropriate isolation procedure.

If for example a biotin-label is used for labelling polypeptides, streptavidine-based chromatographic systems such as streptavidine-labelled or neuravidine-labelled beads as they are commercially available can be used. For His-tags, Ni-NTA agarose as available from Qiagen may be used. For Glutathion-S-transferase labels, GST-sepharose may be used etc.

By subjecting labelled polypeptides from e.g. lysed cells to such chromatographic media, purification of labelled polypeptides can be achieved. As the labelling procedure will typically preferentially label cell surface polypeptides, the fraction retained on and eluted from the chromatographic media may be designated as labelled cell surface polypeptide fraction. The polypeptides found in the flow through may be designated as the intracellular polypeptide fraction. Further, special preparation protocols as they are commonly known may be used to obtain fractions from the lysed cells comprising primarily cell surface associated polypeptides and intracellular polypeptides. Subjecting such fractions to chromatographic media as described above may improve adherence of labelled cell surface polypeptides to the chromatographic media.

If for example cells have been labelled with biotin, polypeptides of the cell will be preferentially labelled over cytoplasmic polypeptides such that labelled cell surface polypeptide will be preferentially retained on e.g. streptavidine-beads while cytoplasmic will be primarily found in the flow through or wash fractions.

The person skilled in the art is aware how to correctly perform these isolation/purification procedures, e.g. how to select suitable washing buffers, elution buffers, pH-values etc.

The labelled polypeptides from the cell surface polypeptide-fraction will thus be isolated by eluting them form the respective chromatographic media.

Subsequently, such isolated labelled polypeptides will be identified. For this, one may use e.g. mass spectrometry (MS) analysis.

The data obtained by this procedure will then be correlated with genomic expression data on genes, the expression of which is up- or down-regulated in CRC. Genomic expression data may be obtained from e.g. Carvalho et al. (2009) Gut 58 (1), 79-89 which is incorporated by reference as far as it provides information on genes the expression of which is upregulated in CRC.

Correlation in the context of the present invention means that only those identified labelled polypeptides will be selected for which genomic expression data indicate an increased expression compared to cells obtained from healthy individuals.

In order to increase the likelihood that the labelled, isolated and identified polypeptides indeed localise to the plasma membrane of cells, a further selection criteria is applied which selects only those polypeptides for which an algorithm-based analysis indicates the presence of a transmembrane (TM) domain and/or at least one signal peptide. The term "signal peptide" refers to sequence signatures that direct localization of proteins to the plasma membrane of cells. Such signal peptide sequences are known in the art.

This analysis may be conducted using programmes and algorithms commonly used for such approaches such as Phobias developed by the Stockholm Bioinformatics Center (Käll et al. (2004) Journal of Molecular Biology, 338(5):1027-1036).

After selection of polypeptides with at least one transmembrane domain or at least one signal peptide, a further selection is applied to reduce the number of false positives. To this end, one selects only such proteins with a positive relative count (RSC) value.

In such an analysis, one determines which labelled polypeptides are enriched in the cell surface polypeptide fraction (i.e. preferentially retained on the chromatographic media) when compared to the intracellular polypeptide fraction. A log2 ratio measured from spectral counts (RSC) analysis is then performed. In principle, this analysis relies on a label-free quantification approach based on spectral counts and is suitable to calculate relative protein abundance between samples. A detailed description can be found in Old et al (2005) Molecular & Cellular Proteomics 4(10): 1487-1502 which is incorporated by reference as far as the determination of RSC values is concerned.

Using this analysis, only proteins with a positive RSC value are then selected.

The polypeptides identified by this series of different steps are then further analysed by identifying those polypeptides found within at least 70% of cells tested in step a). As pointed out above, the cells may be either obtained from different human individuals or may be established CRC cell lines. Typically, one will analyse at least five different cells meaning that e.g. at least cells from five different human individuals all suffering from CRC or at least five different CRC cell lines are analysed.

In a last selection step, polypeptides are selected for which histological analysis confirms localisation at least to the plasma membrane. Such histological analysis may be undertaken using e.g. antibodies being specific for the selected polypeptides.

Typically, the histological analysis will involve immunofluorescent analysis of cells obtained either from human individuals suffering from CRC or of established CRC cell lines. The specific protocols used for such histological analysis will usually depend on the specific polypeptide analysed. Nevertheless, the person skilled in the art will be able to rely on knowledge generally available for e.g. immunofluorescent detection and localisation of polypeptides.

Basically, such approaches will involve fixation of cells on a solid support such as a transparent microscopic cover slide. Subsequently, the cells are lysed and fixed before labelling with e.g. antibodies is performed.

Such approaches are described *inter alia* in Carvalho et al. (2009) Gut 58 (1), 79-89.

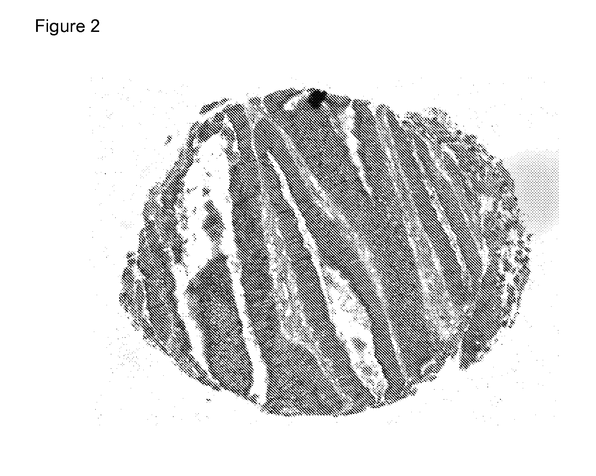

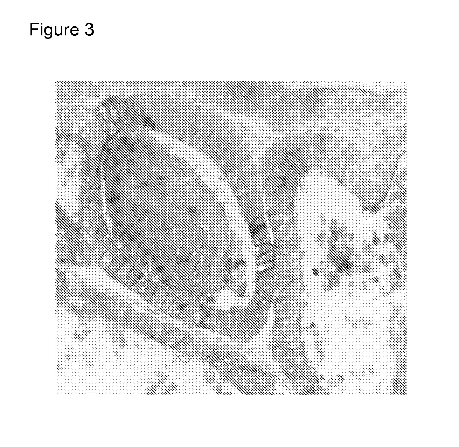

The inventors of the present invention relying on the steps a) to step h) have succeeded in identifying a set of 32 proteins which fulfil the aforementioned criteria. Further, histological analysis of PRNP (SEQ ID NO: 6) and BCAM (SEQ ID NO: 5) confirmed that these two proteins indeed localise at least to the plasma membrane (see Figure 2 and Figure 3, respectively).

A summary of the properties of these 32 proteins can be taken from Table 1.

**Table 1**

| | | | | | | |
|---|---|---|---|---|---|---|
| PLXNA1 Plexin-A1 precursor | 1 | 1 | 211071,9 | 5 | 5,972006932 | -0,616236226 |
| SLC1A5 Neutral amino acid transporter B | 2 | 9 | 56581,8 | 5 | 2,653686031 | -0,292097734 |
| SCARB1 Isoform 1 of Scavenger receptor class B member 1 | 3 | 1 | 56956,6 | 5 | 4,256484988 | -0,139340706 |
| ICAM1 Intercellular adhesion molecule 1 precursor | 4 | 1 | 57806,5 | 5 | 4,688794738 | -0,502549651 |
| BCAM Lutheran blood group glycoprotein precursor | 5 | 1 | 67385,9 | 5 | 6,546313113 | -0,511112343 |
| PRNP Major prion protein precursor | 6 | 2 | 27642,7 | 5 | 4,756670474 | -0,549604991 |
| DAG1 Dystroglycan precursor | 7 | 1 | 97563,3 | 5 | 6,54021969 | -0,248052408 |
| PTGFRN Prostaglandin F2 receptor negative regulator precursor | 8,32 | 1 | 98537,5 | 4 | 6,027860921 | -0,298927655 |
| IGSF8 Isoform 1 of Immunoglobulin superfamily member 8 precursor | 9 | 1 | 65014,8 | 5 | 4,376764152 | -0,32307837 |
| LDLR Low-density lipoprotein receptor precursor | 10 | 1 | 95356,7 | 5 | 5,046917378 | -0,409453599 |
| SLC1A3 Excitatory amino acid transporter 1 | 11 | 7 | 59555,8 | 4 | 3,673690871 | -0,56633461 |
| ACSL1 Isoform 1 of Long-chain-fatty-acid--CoA ligase 1 | 12 | 1 | 77927,1 | 4 | 2,494870467 | -0,468910389 |
| ITFG3 69 kDa protein | 13 | 1 | 69329,2 | 5 | 3,369073363 | -0,537911898 |
| LRP8 Isoform 1 of Low-density lipoprotein receptor-related protein 8 precursor | 14 | 1 | 105658,4 | 4 | 1,495012337 | -0,447612397 |
| CYB5B cytochrome b5 outer mitochondrial membrane precursor | 15 | 1 | 16676,8 | 5 | 3,31042916 | -0,40857396 |
| COPS6 COP9 signalosome complex subunit 6 | 16 | 1 | 36145,4 | 5 | 1,0369939 | -0,243432062 |
| PRSS8 Prostasin precursor | 17 | 2 | 36745,6 | 4 | 4,462248084 | -0,603233346 |
| SDC4 Syndecan-4 precursor | 18 | 1 | 21624,1 | 4 | 2,331824104 | -0,636360718 |
| SLC2A1 Solute carrier family 2, facilitated glucose transporter member 1 | 19 | 12 | 54067,3 | 5 | 2,144242719 | -0,65733993 |
| BST2 Bone marrow stromal antigen 2 precursor | 20 | 1 | 19751,3 | 4 | 3,045513157 | -0,902767067 |
| HSD17B7 Isoform 1 of 3-keto-steroid reductase | 21 | 1 | 38189,9 | 5 | 2,505469898 | -0,19805186 |
| CPOX Coproporphyrinogen III oxidase, mitochondrial precursor | 22 | 1 | 50133,9 | 5 | 1,673454923 | -0,22411626 |
| PODXL Podocalyxin-like protein 1 precursor | 23 | 1 | 55577,7 | 4 | 2,807508469 | -0,376649598 |
| DHCR7 7-dehydrocholesterol reductase | 24 | 7 | 54473 | 5 | 1,858119548 | -0,350599051 |
| SLC4A2 Isoform A of Anion exchange protein 2 | 25 | 13 | 136994,4 | 4 | 4,5335838 | -0,284851412 |
| CNNM3 cyclin M3 isoform 1 | 26 | 3 | 76102,5 | 4 | 2,904753941 | -0,126520099 |
| SLC7A11 Cystine/glutamate transporter | 27 | 14 | 55407,5 | 5 | 3,275370303 | -0,20939536 |
| KIAA1524 Isoform 1 of Protein KIAA1524 | 28 | 1 | 102171,3 | 4 | 2,419056717 | -0,281066707 |
| AUP1 Isoform Long of Ancient ubiquitous protein 1 precursor | 29 | 2 | 53010,8 | 4 | 2,526552401 | -0,187455693 |
| SLC1A4 Neutral amino acid transporter A | 30 | 9 | 55706,4 | 4 | 2,369591427 | -0,186930822 |
| TMEM16F Transmembrane protein 16F | 31 | 8 | 106151,4 | 5 | 3,310893761 | -0,205564206 |

| | | | | | | |
|---|---|---|---|---|---|---|
| "Tm" indicates the number of transmembrane domains."Protein MW" indicates the theoretic molecular weight."Cell line count" indicates in how many of the five CRC cell lines Colo205, Caco2, HT29, RKO and HCT 116 the respective polypeptide was observed. "RSC" indicates the RSC value measured. "DIFF IN EXPRESSION" indicates the difference by which the gene is overexpressed in CRC based on the data of Carvalho et al. (2009) *Gut* 58 (1). | | | | | | |

By applying even stricter selection criteria (namely using an RSC of at least 2.5, a set of 11 proteins emerged which may be particularly useful as diagnostic markers, or targets for contrast agents. This list of 11 polypeptides is shown in Table 2.

**Table 2**

| | | | | | | |
|---|---|---|---|---|---|---|
| PLXNA1 Plexin-A1 precursor | 1 | 1 | 211071,9 | 5 | 5,972006932 | -0,616236226 |
| SLC1A5 Neutral amino acid transporter B | 2 | 9 | 56581,8 | 5 | 2,653686031 | -0,292097734 |
| SCARB 1 Isoform 1 of Scavenger receptor class B member 1 | 3 | 1 | 56956,6 | 5 | 4,256484988 | -0,139340706 |
| ICAM1 Intercellular adhesion molecule 1 precursor | 4 | 1 | 57806,5 | 5 | 4,688794738 | -0,502549651 |
| BCAM Lutheran blood group glycoprotein precursor | 5 | 1 | 67385,9 | 5 | 6,546313113 | -0,511112343 |
| PRNP Major prion protein precursor | 6 | 2 | 27642,7 | 5 | 4,756670474 | -0,549604991 |
| DAG1 Dystroglycan precursor | 7 | 1 | 97563,3 | 5 | 6,54021969 | -0,248052408 |
| PTGFRN Prostaglandin F2 receptor negative regulator precursor | 8,32 | 1 | 98537,5 | 4 | 6,027860921 | -0,298927655 |
| IGSF8 Isoform 1 of Immunoglobulin superfamily member 8 precursor | 9 | 1 | 65014,8 | 5 | 4,376764152 | -0,32307837 |
| LDLR Low-density lipoprotein receptor precursor | 10 | 1 | 95356,7 | 5 | 5,046917378 | -0,409453599 |

| | | | | | | |
|---|---|---|---|---|---|---|
| Tm" indicates the number of transmembrane domains."Protein MW" indicates the theoretic molecular weight."Cell line count" indicates in how many of the five CRC cell lines Colo205, Caco2, HT29, RKO and HCT 116 the respective polypeptide was observed. "RSC" indicates the RSC value measured. "DIFF IN EXPRESSION" indicates the difference by which the gene is overexpressed in CRC based on the data of Carvalho et al. (2009) Gut 58 (1) (***Is this correct? YES***). | | | | | | |

Accordingly, one embodiment of the present invention relates to a diagnostic marker, preferably for detecting CRC comprising at least one polypeptide of the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31; and/or
- A polypeptide of SEQ ID No.: 32.

The term "diagnostic marker" refers to the property of polypeptides of SEQ ID No. 1 to 32 as being suitable as a marker for detecting cancers such as CRC development. Thus, detection of at least one polypeptide of SEQ ID No. 1 to SEQ ID No. 32 will, in principle, allow one to decide whether a human individual for which over-expression of such a polypeptide in comparison to a suitable control has been shown suffers from e.g. CRC.

The person skilled in the art will be aware that the simultaneous detection of more than at least one polypeptide of SEQ ID No. 1 to 32 will increase the correctness of the prediction for the occurrence of e.g. CRC.

The present invention therefore also relates in a preferred embodiment to a diagnostic marker or a set of diagnostic markers comprising at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty one, at least twenty two, at least twenty three, at least twenty four, at least twenty five, at least twenty six, at least twenty seven, at least twenty eight, at least twenty nine, at least thirty or thirty one polypeptides of the group consisting of SEQ ID Nos. 1 to 32.

A preferred diagnostic marker in accordance with the present invention relates to a diagnostic marker or a set of diagnostic markers comprising at least ten polypeptides of the group consisting of SEQ ID No. 1 to 10 and SEQ ID No. 32.

A further preferred diagnostic marker relates to a diagnostic marker or a set of diagnostic markers comprising at least the polypeptides of SEQ ID Nos. 1, 2 and 3.

Yet another preferred diagnostic marker of the present invention relates to a diagnostic marker or a set of diagnostic markers comprising at least the polypeptides of SEQ ID No. 1, 2, 3 and 4.

The present invention also relates to the use of at least one polypeptide selected from the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31; and/or
- A polypeptide of SEQ ID No.: 32;
as a diagnostic marker for detecting CRC.

The present invention also relates to the use of at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty one, at least twenty two, at least twenty three, at least twenty four, at least twenty five, at least twenty six, at least twenty seven, at least twenty eight, at least twenty nine, at least thirty or thirty one polypeptides of the group consisting of SEQ ID Nos. 1 to 32 as diagnostic markers for detecting CRC.

Preferably the invention relates to the use of at least ten polypeptides of the group consisting of SEQ ID No. 1 to 10 and SEQ ID No.: 32 as a diagnostic marker for detecting CRC.

Another preferred embodiment relates to the use of the polypeptides of SEQ ID Nos. 1, 2 and 3 as a diagnostic marker for detecting CRC.

Yet another preferred embodiment relates to to the use of the polypeptides of SEQ ID Nos. 1, 2, 3 and 4 as a diagnostic marker for detecting CRC.

In a preferred embodiment, the polypeptides selected from the group consisting of SEQ ID No. 1 to SEQ ID No. 32 and the above mentioned preferred combinations of polypeptides such as e.g. polypeptides with SEQ ID Nos. 1 to 10 and SEQ ID No.: 32, polypeptides of SEQ ID Nos. 1, 2 and 3, or polypeptides of SEQ ID Nos. 1, 2, 3 and 4 will be used in a diagnostic approach outside the human or animal body.

Another embodiment of the present invention uses at least one polypeptide selected from the group consisting of SEQ ID Nos: 1 to 32 or at least the aforementioned preferred combinations in a diagnostic approach that allows for online detection of the diagnostic marker within a human individual. Such a diagnostic approach may rely on magnetic resonance imaging (MRI) and/or magnetic photon resonance imaging (MPI). However, other approaches which allow the imaging of the presence of at least one polypeptide selected from the group consisting of SEQ ID Nos. 1 to 32 in order to detect CRC may also be applied.

Thus, in one embodiment the present invention relates to a contrast agent being capable of specifically detecting at least polypeptide selected from the group consisting of SEQ ID Nos. 1 to 32.

The term "contrast agent" and its grammatical variations refers to a molecular compound that is capable of specifically interacting with a polypeptide of SEQ ID Nos. 1 to 32 and which can be detected by an apparatus positioned outside the human or animal body. Preferably, such contrast agents are suitable for use in magnetic resonance imaging (MRI) or magnetic photon imaging (MPI).

The term "specifically interacting" and its grammatical variations refer to the property of a molecular compound to preferentially interact with a polypeptide of SEQ ID Nos. 1 to 32 on the cell surface of cells being present within the human or animal body over other proteins that are expressed by such cells.

Preferred contrast agents which may also be designated as contrast agent compositions will be capable of specifically detecting at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty one, at least twenty two, at least twenty three, at least twenty four, at least twenty five, at least twenty six, at least twenty seven, at least twenty eight, at least twenty nine, at least thirty or thirty one polypeptides of the group consisting of SEQ ID Nos. 1 to 32. Other preferred contrast agents/compositions will be capable of detecting the group of polypeptides consisting of SEQ ID No.: 1 to 10 and SEQ ID No.: 32, the group of SEQ ID Nos. 1, 2 and 3 or the group of SEQ ID Nos. 1, 2, 3 and 4.
As MRI and MPI in principle allow for non-invasive molecular imaging, contrast agents which comprise compounds that are capable of specifically interacting with at least one polypeptide of SEQ ID Nos. 1 to 32 and preferably with the aforementioned groups and which can be detected by MRI and/or MPI are preferred.

A class of molecules which may be particularly suitable as contrast agents for the purposes of the present invention are antibodies.

Antibodies being specific for polypeptides of SEQ ID Nos. 1 to 32 and preferably for the aforementioned groups of polypeptides may be already commercially available such as Primary anti-prion, mouse clone 8H4 antibody from Sigma-Aldrich (St. Louis,MO,USA) (for SEQ ID No.6), mouse anti human CD239 from abD serotech (Oxford,UK) (for SEQ ID No.5).

If antibodies specifically recognising polypeptides of SEQ ID Nos. 1 to 32 are not already available, they can be produced by methods known to the person skilled in the art. Such antibodies may be polyclonal or monoclonal antibodies. Monoclonal antibodies may be produced by classical hybridoma fusion technology or e.g. phage-display systems.

The term "antibody" is used in the context of the present invention in its common sense. However, in addition the term further includes antibody variants and derivatives such as a single chain antibody, a Fab-fragment, a Fab2-fragment, a multispecific antibody, a diabody, a triabody, a tetrabody, a minibody, a linear antibody, a chelating recombinant antibody, a tribody, a bibody, an intrabody, a nanobody, a small modular immunopharmaceutical (SMIP), a binding-domain immunoglobulin fusion protein, a camelized antibody, a V_{HH} containing antibody and the like.

Preferred embodiments of the present invention relate to contrast agents which comprise antibodies that interact at least with polypeptides of SEQ ID Nos. 1 to 10 and SEQ ID No.: 32, with polypeptides of SEQ ID Nos. 1, 2 and 3 or with polypeptides of SEQ ID Nos. 1, 2, 3 and 4.

Contrast agents comprising such antibodies may be provided in a form where the contrast agent is a composition comprising different sets of antibody with each antibody recognising a polypeptide of a specific SEQ ID. Thus, a contrast agent may comprise three antibodies recognising polypeptides of SEQ ID Nos. 1, 2 and 3, respectively.

However, contrast agents in accordance with the present invention may also comprise antibodies which are multi-specific. Thus, a contrast agent may comprise a single antibody which recognises e.g. the polypeptide of SEQ ID No. 1, 2 and 3, at the same time. Further, contrast agents in accordance with the present invention may comprise antibodies all of which recognise a polypeptide of the same SEQ ID. Thus, a contrast agent in accordance with the present invention may comprise e.g. three antibodies all of which are specific for SEQ ID No. 1 and e.g. four antibodies all of which are specific for SEQ ID No. 2 etc.

Contrast agents, aside from their property of being capable of specifically recognising polypeptides of SEQ ID Nos. 1 to 32 will in addition typically comprise a marker molecule which is detectable by the specific detection technology used.

For example, if fluorescent spectroscopy is used as a detection means, such marker molecules may comprise fluorophores as detectable marker molecules that can be excitated at a specific wavelength.

With respect to preferred contrast agents in accordance with the invention that are suitable for MRI, the contrast agents such as the above described antibodies may comprise a marker molecule which is detectable by MRI. Such detectable labels include e.g. USPIOS and 19Fluor.

Another embodiment of the present invention relates to the use of at least one antibody capable of interacting with a polypeptide selected from the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31; and/or
- A polypeptide of SEQ ID No.: 32 as a contrast agent suitable for MRI.

The invention also relates to the use of antibodies which interact with at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty one, at least twenty two, at least twenty three, at least twenty four, at least twenty five, at least twenty six, at least twenty seven, at least twenty eight, at least twenty nine, at least thirty or thirty one polypeptides of the group consisting of SEQ ID Nos. 1 to 32 as contrast agents.

Preferably the invention relates to the use of antibodies which interact with at least ten polypeptides of the group consisting of SEQ ID No. 1 to 10 and SEQ ID No.: 32 as contrast agents.

Another preferred embodiment relates to the use of antibodies which interact with the polypeptides of SEQ ID Nos. 1, 2 and 3 as contrast agents.

Yet another preferred embodiment relates to the use of antibodies which interact with the polypeptides of SEQ ID Nos. 1, 2, 3 and 4 as contrast agents.

As pointed out above, the diagnostic markers and contrast agents of the present invention may be used for detection and diagnosis of colorectal cancer. These methods may be used either outside or inside the human or animal body. Thus, one may e.g. use an antibody which is labelled to a fluorophore in the histological analysis for detecting polypeptides of SEQ ID Nos. 1 to 32 in cellular tissue and samples which have been obtained from an individual suspected of suffering from CRC.

In addition or alternatively, one may use e.g. antibodies for non-invasive molecular imaging techniques such as MRI.

As the diagnostic markers and contrast agents as described above are primarily used for the detection and diagnosis of CRC, one embodiment of the present invention relates to a method of diagnosing CRC comprising at least the following steps:
a) Obtaining at least one sample from at least one human or animal individual suspected to suffer from ongoing or imminent CRC development;
b) Testing in said at least one sample for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
c) Testing in at least one control sample obtained from at least one human or animal individual not suffering from ongoing or imminent CRC development for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31
   - A polypeptide of SEQ ID No.: 32;
d) Determining difference in expression of steps b) and d);
e) Deciding on the presence or imminence of CRC development based on the results obtained in step d).

In one embodiment, steps b), c), d) and/or e) of this method of diagnosis are performed outside the human or animal body.

Another embodiment of the present invention relates to a method of diagnosing CRC comprising at least the following steps:
a) Testing in at least one human or animal individual suspected to suffer from ongoing or imminent CRC development for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31
   - A polypeptide of SEQ ID No.: 32;
b) Comparing expression as determined in step a) with expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
   as determined for human or animal individuals not suffering from ongoing or imminent colorectal cancer development
c) Deciding on the presence or imminence of colorectal cancer development based on the results obtained in step b).

Further, the present invention may relate to a method of data acquisition comprising at least the following steps:
a) Testing in at least one human or animal individual suspected to suffer from ongoing or imminent CRC development for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
b) Comparing expression as determined in step a) with expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
as determined for human or animal individuals not suffering from ongoing or imminent CRC development

The afore-described methods have in common that they determine the occurrence of at least one polypeptide of SEQ ID Nos. 1 to 32 either in test samples obtained from human or animal individuals being suspected of suffering from CRC development or directly in human or animal individuals being suspected of suffering from CRC development.

Further, the data obtained for these molecules are then compared with the data obtained either from control samples or control individuals. The comparison with the control samples or control individuals serves to identify these test samples obtained from human individuals being suspected of suffering from CRC or to identify these human individuals being suspected of suffering from CRC in which at least one polypeptide of SEQ ID Nos. 1 to 32 is over-expressed compared to the respective control sample or control individual.

The term "control sample" or "control individual" therefore refers either to a sample obtained from e.g. a human individual or to e.g. a human individual not suffering from CRC cancer. Such individuals may be identified by performing classical CRC diagnosis. A person skilled in the art will be aware that for the purposes of the comparison between a test sample versus control sample and a test individual versus control individual, a proper standardization of the obtained expression data must be undertaken. However, such standardization is common in the art and usually includes determination of expression of polypeptides of SEQ ID Nos. 1 to 32 in relation to e.g. a polypeptide not being involved in colorectal cancer. Thus, in one aspect, data obtained from test samples and control samples may be standardized with respect to expression of a compound such as e.g. actine.

In preferred embodiments of the above mentioned methods of diagnosis or data acquisition, one monitors expression of at least the ten polypeptides of SEQ ID No.1 to 10 and SEQ ID No.: 32, of the polypeptides of SEQ ID No. 1, 2, 3 and 4, or of the polypeptides of SEQ ID No. 1, 2 and 3.

In the following, the present invention will be described with respect to some specific examples. These examples are however not to be construed as being limiting.

### EXAMPLES

### Experiment 1: Identification of cell surface proteins potentially involved in colorectal cancer

Colorectal cancer (CRC) cell lines COLO 205, HT-29, Caco-2, RKO and HCT116 were obtained from the American Type Culture Collection (ATCC). These are all cell lines used as model system for CRC (Lengauer. et al (1997) PNAS 94(6):2545-2550).

All cells except CACO2 were grown in complete medium (Dulbecco's Modified Eagle's Medium, DMEM; Lonza Biowhittaker, Verviers, Belgium) containing 10% Fetal Calf Serum (FCS) and 1% Penicillin/Streptomycin (penicillin 50 units/ml, Astellas Pharma B.V., Leiderdorp, Netherlands; streptomycin 50ug/ml, Fisiopharma, Palomonta (SA), Italy) at 37°C with a CO₂ atmosphere concentration of 5%. The CACO2 cell line was grown in RPMI1640 (Lonza Biowhittaker, Verviers, Belgium) containing 20% of FCS and 1% Penicillin/Streptomycin (penicillin 50 units/ml, Astellas Pharma B.V., Leiderdorp, Netherlands); streptomycin 50ug/ml (Fisiopharma, Palomonta (SA), Italy) at 37°C with a CO₂ atmosphere concentration of 5%.

The cell lines were grown until a confluency of 70/80% was obtained.

Subsequently, the cells were labelled with biotin by incubating the cells at 4°C for 30 min with 412µM Sulfo-NHS-SS-Biotin (Pierce, Rockford, USA) dissolved in PBS.

Following this incubation the cells were washed with PBS and lysed in lysis buffer (Pierce) supplemented with protease inhibitor cocktail (pic) (Complete Protease Inhibitor Cocktail, 1x, Roche, Mannheim, Germany)

The cell lysate was incubated at 4°C for 120 min with NeutrAvidin Protein beads (Pierce) in a rotator to achieve binding of the biotin labelled proteins to the beads. The beads were than washed in two wash buffers. First three times in wash buffer A containing 1% w/v nonidet P-40 and 0.1% w/v SDS in PBS followed by three times in wash buffer B containing 0.1% w/v nonidet P-40 and 0.5 M NaCl in PBS.

The proteins were than eluted from the beads using PBS containing 50 mM DTT and 62.5 mM Tris HCl. This fraction was designated as cell surface polypeptide fraction.

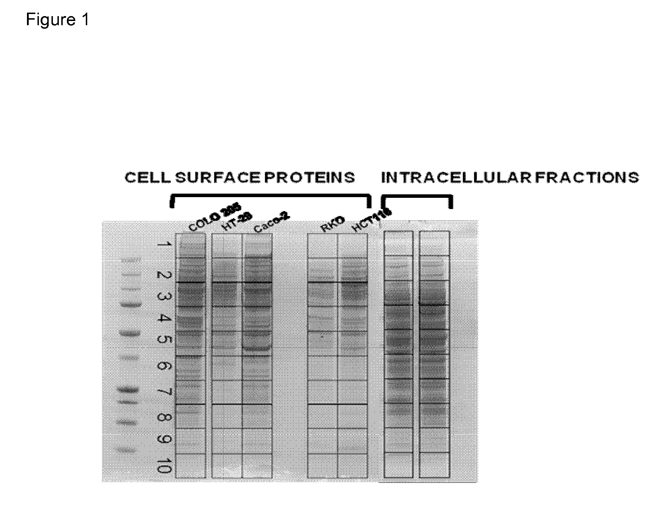

Subsequently, the purified fractions were analysed by SDS-PAGE. An example of biotin-labelled cell surface and intracellular fractions is shown in Fig. 1.

The streptavidine-purified biotin-labelled proteins of the cell surface fraction were then identified by mass spectrometry.

After electrophoresis the gels were fixed in 50% ethanol containing 3% phosphoric acid and stained with Coomassie R-250. After staining the gels were washed in MilliQ water and stored at 4 °C until processing for in-gel digestion.

The gel lanes corresponding to the cell surface proteins of the five different cell lines and the mixtures of intracellular fractions were cut in 10 bands. Each band was processed for in-gel digestion. Briefly, bands were washed dehydrated three times in 50 mM ABC (ammonium bicarbonate pH 7.9)/50 mM ABC + 50% ACN (acetonitrile).

Subsequently, cysteine bonds were reduced with 10mM dithiotreitol for 1h at 56°C and alkylated with 50 mM iodoacetamide for 45 min at RT in the dark. After two subsequent wash/dehydration cycles the bands were dried 10 min in a vacuum centrifuge and incubated overnight with 0.06 µg/µl trypsin at 25 °C. Peptides were extracted once in 1% formic acid and

Subsequently two times in 50% ACN in 5% formic acid. The volume was reduced to 50 µl in a vacuum centrifuge prior to LC-MS/MS analysis.

Peptides were separated by an Ultimate 3000 nanoLC system (Dionex LC-Packings, Amsterdam, The Netherlands) equipped with a 20 cm×75 µm ID fused silica column custom packed with 3 µm 100 Å ReproSil Pur C18 aqua (Dr Maisch GMBH, Ammerbuch-Entringen, Germany). After injection, peptides were trapped at 30 µl/min on a 5 mm×300 µm ID Pepmap C18 cartridge (Dionex LC-Packings, Amsterdam, The Netherlands) at 2% buffer B (buffer A: 0.05% formic acid in MQ; buffer B: 80% ACN+0.05% formic acid in MQ) and separated at 300 nl/min in a 10-40% buffer B gradient in 60 min.

Eluting peptides were ionized at 1.7 kV in a Nanomate Triversa Chip-based nanospray source using a Triversa LC coupler (Advion, Ithaca, NJ). Intact peptide mass spectra and fragmentation spectra were acquired on a LTQ-FT hybrid mass spectrometer (Thermo Fisher, Bremen, Germany). Intact masses were measured at resolution 50.000 in the ICR cell using a target value of 1×10⁶ charges. In parallel, following an FT pre-scan, the top 5 peptide signals (charge-states 2+ and higher) were submitted to MS/MS in the linear ion trap (3 amu isolation width, 30 ms activation, 35% normalized activation energy, Q value of 0.25 and a threshold of 5000 counts). Dynamic exclusion was applied with a repeat count of 1 and an exclusion time of 30 s.

MS/MS spectra were searched against the human IPI database 3.31(67511 entries) using Sequest (version 27, rev 12), which is part of the BioWorks 3.3 data analysis package (Thermo Fisher, San Jose, CA). MS/MS spectra were searched with a maximum allowed deviation of 10 ppm for the precursor mass and 1 amu for fragment masses. Methionine oxidation and cysteine carboxamidomethylation were allowed as variable modifications. Two missed cleavages were allowed and the minimum number of tryptic termini was 1. After database searching the DTA and OUT files were imported into Scaffold 1.07 (Proteome software, Portland, OR). Scaffold was used to organize the gelband data and to validate peptide identifications using the Peptide Prophet algorithm [14], only identifications with a probabilityN95% were retained. Subsequently, the Protein

Prophet algorithm [15] was applied and protein identifications with a probability of N99% with 2 peptides or more in at least one of the samples were retained. Proteins that contained similar peptides and could not be differentiated based on MS/MS analysis alone were grouped. For each protein identified, the number of unique peptides was exported to Excel. (Piersma et al. (2009) J. Proteomics 72(1): 91-109)

This analysis revealed approximately 2986 proteins.

In parallel, an analysis of genes being implicated in the transgression of adenoma to colorectal adenocarcinoma was performed. This analysis revealed approximately 2478 genes the expression of which is either up- or down-regulated. This approach is described in detail in Carvalho et al. in Gut (2009), 58(1):79-89.

Based on this analysis, only those proteins of the purified cell surface fraction for which the gene analysis data revealed an over-expression were selected. This led to identification of approximately 1305 proteins. Of these 355 genes were over expressed with a significant p-value (>0.05) according to the Wilcox rank test (see Carvalho et al Gut (2009), 58(1):79-89).

In a next step, proteins were selected which comprised at least one transmembrane (TM) domain.

To this end, the software program Phobias developed by the Stockholm Bioinformatics Center was used. (Käll et al. (2004) Journal of Molecular Biology, 338(5):1027-1036). This program indicates whether a protein contains a transmembrane region and/or a signal peptide. Proteins containing either one of these or both were selected.

To reduce the number of false positives we determined which proteins were enriched in the cell surface fractions when compared to the non-biotinylated fractions. A log2 ratio measured from spectral counts (RSC) analysis was carried out. This analysis relied on a label-free quantification approach based on spectral counts. Such an approach is well suited to calculate relative protein abundance between samples and was done according to the method of Old et al. (Old et al (2005) Molecular & Cellular Proteomics 4(10): 1487-1502).

Using this analysis, only proteins with a positive RSC value were selected.

Finally, proteins were selected which were found in at least 70% of the CRC cell lines tested.

This revealed a set of 31 proteins having SEQ ID Nos.: 1 to 32.

A further set of 11 proteins was identified for which the RSC value increased at least by a factor of 2.5. These proteins have SEQ ID Nos. 1 to 10 and SEQ ID No.: 32.

Of these, histological analysis was performed for PRNP (SEQ ID No.: 6) and BCAM (SEQ ID No.: 5) on tumor material obtained from CRC patients.

A 4 mm section of formalin fixed paraffin embedded (FFPE) tissue was used for immunohistochemistry. After deparaffination in xylene, and rehydration through graded alcohol to water, endogenous peroxidase was blocked with hydrogen peroxide (0.3% H2O2/methanol) for 30 min. Antigen retrieval was done by autoclaving in 1.5mM HCL for PRNP and TRIS/EDTA ph 9 buffer for BCAM. Primary anti-prion mouse clone 8H4 antibody from sigma-aldrich (St. Louis,MO,USA) was incubated overnight at a dilution of 1:100 and mouse anti human CD239 from abD serotech (Oxford,UK) was also incubated overnight at a dilution of 1:100. Following extensive washing the slides were incubated for 30 min with poly-HRP-goat anti-mouse/rabbit/rat IgG (Powervision, Immunologic, Duiven). Then all sections were washed and incubated with DAB Substrate-Chromogen (Dako, Glostrup, Denmark). Counterstaining was done with Mayer's haematoxylin. Incubation without primary antibody was used as negative control.

The results can be seen in Fig. 2 for PRNP and Fig. 3 for BCAM.

## Claims

1. Diagnostic marker comprising at least one polypeptide of the group consisting of:
• A polypeptide of SEQ ID No.: 1;
• A polypeptide of SEQ ID No.: 2;
• A polypeptide of SEQ ID No.: 3;
• A polypeptide of SEQ ID No.: 4;
• A polypeptide of SEQ ID No.: 5;
• A polypeptide of SEQ ID No.: 6;
• A polypeptide of SEQ ID No.: 7;
• A polypeptide of SEQ ID No.: 8;
• A polypeptide of SEQ ID No.: 9;
• A polypeptide of SEQ ID No.: 10;
• A polypeptide of SEQ ID No.: 11;
• A polypeptide of SEQ ID No.: 12;
• A polypeptide of SEQ ID No.: 13;
• A polypeptide of SEQ ID No.: 14;
• A polypeptide of SEQ ID No.: 15;
• A polypeptide of SEQ ID No.: 16;
• A polypeptide of SEQ ID No.: 17;
• A polypeptide of SEQ ID No.: 18;
• A polypeptide of SEQ ID No.: 19;
• A polypeptide of SEQ ID No.: 20;
• A polypeptide of SEQ ID No.: 21;
• A polypeptide of SEQ ID No.: 22;
• A polypeptide of SEQ ID No.: 23;
• A polypeptide of SEQ ID No.: 24;
• A polypeptide of SEQ ID No.: 25;
• A polypeptide of SEQ ID No.: 26;
• A polypeptide of SEQ ID No.: 27;
• A polypeptide of SEQ ID No.: 28;
• A polypeptide of SEQ ID No.: 29;
• A polypeptide of SEQ ID No.: 30;
• A polypeptide of SEQ ID No.: 31; and/or
• A polypeptide of SEQ ID No.: 32.

2. Diagnostic marker of claim 1 comprising at least the polypeptides:
• A polypeptide of SEQ ID No.: 1;
• A polypeptide of SEQ ID No.: 2; and/or
• A polypeptide of SEQ ID No.: 3.

3. Use of at least one polypeptide selected from the group consisting of:
• A polypeptide of SEQ ID No.: 1;
• A polypeptide of SEQ ID No.: 2;
• A polypeptide of SEQ ID No.: 3;
• A polypeptide of SEQ ID No.: 4;
• A polypeptide of SEQ ID No.: 5;
• A polypeptide of SEQ ID No.: 6;
• A polypeptide of SEQ ID No.: 7;
• A polypeptide of SEQ ID No.: 8;
• A polypeptide of SEQ ID No.: 9;
• A polypeptide of SEQ ID No.: 10;
• A polypeptide of SEQ ID No.: 11;
• A polypeptide of SEQ ID No.: 12;
• A polypeptide of SEQ ID No.: 13;
• A polypeptide of SEQ ID No.: 14;
• A polypeptide of SEQ ID No.: 15;
• A polypeptide of SEQ ID No.: 16;
• A polypeptide of SEQ ID No.: 17;
• A polypeptide of SEQ ID No.: 18;
• A polypeptide of SEQ ID No.: 19;
• A polypeptide of SEQ ID No.: 20;
• A polypeptide of SEQ ID No.: 21;
• A polypeptide of SEQ ID No.: 22;
• A polypeptide of SEQ ID No.: 23;
• A polypeptide of SEQ ID No.: 24;
• A polypeptide of SEQ ID No.: 25;
• A polypeptide of SEQ ID No.: 26;
• A polypeptide of SEQ ID No.: 27;
• A polypeptide of SEQ ID No.: 28;
• A polypeptide of SEQ ID No.: 29;
• A polypeptide of SEQ ID No.: 30;
• A polypeptide of SEQ ID No.: 31; and/or
• A polypeptide of SEQ ID No.: 32.
as a diagnostic marker.

4. Use of claim 3, wherein at least the following polypeptides are used:
• A polypeptide of SEQ ID No.: 1;
• A polypeptide of SEQ ID No.: 2; and
• A polypeptide of SEQ ID No.: 3.

5. A contrast agent, optionally for use in MRI and/or MPI comprising at least one compound being capable of interacting with a polypeptide selected from the group consisting of:
• A polypeptide of SEQ ID No.: 1;
• A polypeptide of SEQ ID No.: 2;
• A polypeptide of SEQ ID No.: 3;
• A polypeptide of SEQ ID No.: 4;
• A polypeptide of SEQ ID No.: 5;
• A polypeptide of SEQ ID No.: 6;
• A polypeptide of SEQ ID No.: 7;
• A polypeptide of SEQ ID No.: 8;
• A polypeptide of SEQ ID No.: 9;
• A polypeptide of SEQ ID No.: 10;
• A polypeptide of SEQ ID No.: 11;
• A polypeptide of SEQ ID No.: 12;
• A polypeptide of SEQ ID No.: 13;
• A polypeptide of SEQ ID No.: 14;
• A polypeptide of SEQ ID No.: 15;
• A polypeptide of SEQ ID No.: 16;
• A polypeptide of SEQ ID No.: 17;
• A polypeptide of SEQ ID No.: 18;
• A polypeptide of SEQ ID No.: 19;
• A polypeptide of SEQ ID No.: 20;
• A polypeptide of SEQ ID No.: 21;
• A polypeptide of SEQ ID No.: 22;
• A polypeptide of SEQ ID No.: 23;
• A polypeptide of SEQ ID No.: 24;
• A polypeptide of SEQ ID No.: 25;
• A polypeptide of SEQ ID No.: 26;
• A polypeptide of SEQ ID No.: 27;
• A polypeptide of SEQ ID No.: 28;
• A polypeptide of SEQ ID No.: 29;
• A polypeptide of SEQ ID No.: 30;
• A polypeptide of SEQ ID No.: 31; and/or
• A polypeptide of SEQ ID No.: 32.

6. Contrast agent of claim 5, wherein the contrast agent comprises a compound/compounds which interacts/interact at least with:
• A polypeptide of SEQ ID No.: 1;
• A polypeptide of SEQ ID No.: 2; and
• A polypeptide of SEQ ID No.: 3.

7. Contrast agent of claim 5 or 6, wherein the compound/compounds is/are selected from antibodies which specifically interact with at least one polypeptide of SEQ ID No. 1 to 32.

8. Contrast agent of any of claims 5 to 7, wherein the compound/compounds is/are coupled to a detectable marker molecule.

9. Use of at least one antibody capable of interacting with a polypeptide selected from the group consisting of:
• A polypeptide of SEQ ID No.: 1;
• A polypeptide of SEQ ID No.: 2;
• A polypeptide of SEQ ID No.: 3;
• A polypeptide of SEQ ID No.: 4;
• A polypeptide of SEQ ID No.: 5;
• A polypeptide of SEQ ID No.: 6;
• A polypeptide of SEQ ID No.: 7;
• A polypeptide of SEQ ID No.: 8;
• A polypeptide of SEQ ID No.: 9;
• A polypeptide of SEQ ID No.: 10;
• A polypeptide of SEQ ID No.: 11;
• A polypeptide of SEQ ID No.: 12;
• A polypeptide of SEQ ID No.: 13;
• A polypeptide of SEQ ID No.: 14;
• A polypeptide of SEQ ID No.: 15;
• A polypeptide of SEQ ID No.: 16;
• A polypeptide of SEQ ID No.: 17;
• A polypeptide of SEQ ID No.: 18;
• A polypeptide of SEQ ID No.: 19;
• A polypeptide of SEQ ID No.: 20;
• A polypeptide of SEQ ID No.: 21;
• A polypeptide of SEQ ID No.: 22;
• A polypeptide of SEQ ID No.: 23;
• A polypeptide of SEQ ID No.: 24;
• A polypeptide of SEQ ID No.: 25;
• A polypeptide of SEQ ID No.: 26;
• A polypeptide of SEQ ID No.: 27;
• A polypeptide of SEQ ID No.: 28;
• A polypeptide of SEQ ID No.: 29;
• A polypeptide of SEQ ID No.: 30;
• A polypeptide of SEQ ID No.: 31; and/or
• A polypeptide of SEQ ID No.: 32
as a contrast agent, optionally for use MRI and/or MPI.

10. Use of claim 9, wherein the antibody/antibodies is/are selected from commercially available antibodies which interact with the said polypeptides.

11. Use of claim 9 or 10, wherein the antibody/antibodies is/are coupled to a detectable marker molecule.

12. Method of diagnosing colorectal cancer (CRC) comprising at least the following steps:
a) Obtaining at least one sample from at least one human or animal individual suspected to suffer from ongoing or imminent CRC development;
b) Testing in said at least one sample for expression of at least one polypeptide selected from the group consisting of:
• A polypeptide of SEQ ID No.: 1;
• A polypeptide of SEQ ID No.: 2;
• A polypeptide of SEQ ID No.: 3;
• A polypeptide of SEQ ID No.: 4;
• A polypeptide of SEQ ID No.: 5;
• A polypeptide of SEQ ID No.: 6;
• A polypeptide of SEQ ID No.: 7;
• A polypeptide of SEQ ID No.: 8;
• A polypeptide of SEQ ID No.: 9;
• A polypeptide of SEQ ID No.: 10;
• A polypeptide of SEQ ID No.: 11;
• A polypeptide of SEQ ID No.: 12;
• A polypeptide of SEQ ID No.: 13;
• A polypeptide of SEQ ID No.: 14;
• A polypeptide of SEQ ID No.: 15;
• A polypeptide of SEQ ID No.: 16;
• A polypeptide of SEQ ID No.: 17;
• A polypeptide of SEQ ID No.: 18;
• A polypeptide of SEQ ID No.: 19;
• A polypeptide of SEQ ID No.: 20;
• A polypeptide of SEQ ID No.: 21;
• A polypeptide of SEQ ID No.: 22;
• A polypeptide of SEQ ID No.: 23;
• A polypeptide of SEQ ID No.: 24;
• A polypeptide of SEQ ID No.: 25;
• A polypeptide of SEQ ID No.: 26;
• A polypeptide of SEQ ID No.: 27;
• A polypeptide of SEQ ID No.: 28;
• A polypeptide of SEQ ID No.: 29;
• A polypeptide of SEQ ID No.: 30;
• A polypeptide of SEQ ID No.: 31; and/or
• A polypeptide of SEQ ID No.: 32;
c) Testing in at least one control sample obtained from at least one human or animal individual not suffering from ongoing or imminent CRC development for expression of at least one polypeptide selected from the group consisting of:
• A polypeptide of SEQ ID No.: 1;
• A polypeptide of SEQ ID No.: 2;
• A polypeptide of SEQ ID No.: 3;
• A polypeptide of SEQ ID No.: 4;
• A polypeptide of SEQ ID No.: 5;
• A polypeptide of SEQ ID No.: 6;
• A polypeptide of SEQ ID No.: 7;
• A polypeptide of SEQ ID No.: 8;
• A polypeptide of SEQ ID No.: 9;
• A polypeptide of SEQ ID No.: 10;
• A polypeptide of SEQ ID No.: 11;
• A polypeptide of SEQ ID No.: 12;
• A polypeptide of SEQ ID No.: 13;
• A polypeptide of SEQ ID No.: 14;
• A polypeptide of SEQ ID No.: 15;
• A polypeptide of SEQ ID No.: 16;
• A polypeptide of SEQ ID No.: 17;
• A polypeptide of SEQ ID No.: 18;
• A polypeptide of SEQ ID No.: 19;
• A polypeptide of SEQ ID No.: 20;
• A polypeptide of SEQ ID No.: 21;
• A polypeptide of SEQ ID No.: 22;
• A polypeptide of SEQ ID No.: 23;
• A polypeptide of SEQ ID No.: 24;
• A polypeptide of SEQ ID No.: 25;
• A polypeptide of SEQ ID No.: 26;
• A polypeptide of SEQ ID No.: 27;
• A polypeptide of SEQ ID No.: 28;
• A polypeptide of SEQ ID No.: 29;
• A polypeptide of SEQ ID No.: 30;
• A polypeptide of SEQ ID No.: 31; and/or
• A polypeptide of SEQ ID No.: 32;
d) Determining difference in expression of steps b) and d);
e) Deciding on the presence or imminence of CRC development based on the results obtained in step d).

13. Method of diagnosing CRC comprising at least the following steps:
a) Testing in at least one human or animal individual suspected to suffer from ongoing or imminent CRC development for expression of at least one polypeptide selected from the group consisting of:
• A polypeptide of SEQ ID No.: 1;
• A polypeptide of SEQ ID No.: 2;
• A polypeptide of SEQ ID No.: 3;
• A polypeptide of SEQ ID No.: 4;
• A polypeptide of SEQ ID No.: 5;
• A polypeptide of SEQ ID No.: 6;
• A polypeptide of SEQ ID No.: 7;
• A polypeptide of SEQ ID No.: 8;
• A polypeptide of SEQ ID No.: 9;
• A polypeptide of SEQ ID No.: 10;
• A polypeptide of SEQ ID No.: 11;
• A polypeptide of SEQ ID No.: 12;
• A polypeptide of SEQ ID No.: 13;
• A polypeptide of SEQ ID No.: 14;
• A polypeptide of SEQ ID No.: 15;
• A polypeptide of SEQ ID No.: 16;
• A polypeptide of SEQ ID No.: 17;
• A polypeptide of SEQ ID No.: 18;
• A polypeptide of SEQ ID No.: 19;
• A polypeptide of SEQ ID No.: 20;
• A polypeptide of SEQ ID No.: 21;
• A polypeptide of SEQ ID No.: 22;
• A polypeptide of SEQ ID No.: 23;
• A polypeptide of SEQ ID No.: 24;
• A polypeptide of SEQ ID No.: 25;
• A polypeptide of SEQ ID No.: 26;
• A polypeptide of SEQ ID No.: 27;
• A polypeptide of SEQ ID No.: 28;
• A polypeptide of SEQ ID No.: 29;
• A polypeptide of SEQ ID No.: 30;
• A polypeptide of SEQ ID No.: 31; and/or
• A polypeptide of SEQ ID No.: 32;
b) Comparing expression as determined in step a) with expression of at least one polypeptide selected from the group consisting of:
• A polypeptide of SEQ ID No.: 1;
• A polypeptide of SEQ ID No.: 2;
• A polypeptide of SEQ ID No.: 3;
• A polypeptide of SEQ ID No.: 4;
• A polypeptide of SEQ ID No.: 5;
• A polypeptide of SEQ ID No.: 6;
• A polypeptide of SEQ ID No.: 7;
• A polypeptide of SEQ ID No.: 8;
• A polypeptide of SEQ ID No.: 9;
• A polypeptide of SEQ ID No.: 10;
• A polypeptide of SEQ ID No.: 11;
• A polypeptide of SEQ ID No.: 12;
• A polypeptide of SEQ ID No.: 13;
• A polypeptide of SEQ ID No.: 14;
• A polypeptide of SEQ ID No.: 15;
• A polypeptide of SEQ ID No.: 16;
• A polypeptide of SEQ ID No.: 17;
• A polypeptide of SEQ ID No.: 18;
• A polypeptide of SEQ ID No.: 19;
• A polypeptide of SEQ ID No.: 20;
• A polypeptide of SEQ ID No.: 21;
• A polypeptide of SEQ ID No.: 22;
• A polypeptide of SEQ ID No.: 23;
• A polypeptide of SEQ ID No.: 24;
• A polypeptide of SEQ ID No.: 25;
• A polypeptide of SEQ ID No.: 26;
• A polypeptide of SEQ ID No.: 27;
• A polypeptide of SEQ ID No.: 28;
• A polypeptide of SEQ ID No.: 29;
• A polypeptide of SEQ ID No.: 30;
• A polypeptide of SEQ ID No.: 31; and/or
• A polypeptide of SEQ ID No.: 32;
as determined for human or animal individuals not suffering from ongoing or imminent colorectal cancer development
c) Deciding on the presence or imminence of CRCdevelopment based on the results obtained in step b).

14. Method of data acquisition comprising at least the following steps:
a) Testing in at least one human or animal individual suspected to suffer from ongoing or imminent CRC development for expression of at least one polypeptide selected from the group consisting of:
• A polypeptide of SEQ ID No.: 1;
• A polypeptide of SEQ ID No.: 2;
• A polypeptide of SEQ ID No.: 3;
• A polypeptide of SEQ ID No.: 4;
• A polypeptide of SEQ ID No.: 5;
• A polypeptide of SEQ ID No.: 6;
• A polypeptide of SEQ ID No.: 7;
• A polypeptide of SEQ ID No.: 8;
• A polypeptide of SEQ ID No.: 9;
• A polypeptide of SEQ ID No.: 10;
• A polypeptide of SEQ ID No.: 11;
• A polypeptide of SEQ ID No.: 12;
• A polypeptide of SEQ ID No.: 13;
• A polypeptide of SEQ ID No.: 14;
• A polypeptide of SEQ ID No.: 15;
• A polypeptide of SEQ ID No.: 16;
• A polypeptide of SEQ ID No.: 17;
• A polypeptide of SEQ ID No.: 18;
• A polypeptide of SEQ ID No.: 19;
• A polypeptide of SEQ ID No.: 20;
• A polypeptide of SEQ ID No.: 21;
• A polypeptide of SEQ ID No.: 22;
• A polypeptide of SEQ ID No.: 23;
• A polypeptide of SEQ ID No.: 24;
• A polypeptide of SEQ ID No.: 25;
• A polypeptide of SEQ ID No.: 26;
• A polypeptide of SEQ ID No.: 27;
• A polypeptide of SEQ ID No.: 28;
• A polypeptide of SEQ ID No.: 29;
• A polypeptide of SEQ ID No.: 30;
• A polypeptide of SEQ ID No.: 31; and/or
• A polypeptide of SEQ ID No.: 32;
b) Comparing expression as determined in step a) with expression of at least one polypeptide selected from the group consisting of:
• A polypeptide of SEQ ID No.: 1
• A polypeptide of SEQ ID No.: 2;
• A polypeptide of SEQ ID No.: 3;
• A polypeptide of SEQ ID No.: 4;
• A polypeptide of SEQ ID No.: 5;
• A polypeptide of SEQ ID No.: 6;
• A polypeptide of SEQ ID No.: 7;
• A polypeptide of SEQ ID No.: 8;
• A polypeptide of SEQ ID No.: 9;
• A polypeptide of SEQ ID No.: 10;
• A polypeptide of SEQ ID No.: 11;
• A polypeptide of SEQ ID No.: 12;
• A polypeptide of SEQ ID No.: 13;
• A polypeptide of SEQ ID No.: 14;
• A polypeptide of SEQ ID No.: 15;
• A polypeptide of SEQ ID No.: 16;
• A polypeptide of SEQ ID No.: 17;
• A polypeptide of SEQ ID No.: 18;
• A polypeptide of SEQ ID No.: 19;
• A polypeptide of SEQ ID No.: 20;
• A polypeptide of SEQ ID No.: 21;
• A polypeptide of SEQ ID No.: 22;
• A polypeptide of SEQ ID No.: 23;
• A polypeptide of SEQ ID No.: 24;
• A polypeptide of SEQ ID No.: 25;
• A polypeptide of SEQ ID No.: 26;
• A polypeptide of SEQ ID No.: 27;
• A polypeptide of SEQ ID No.: 28;
• A polypeptide of SEQ ID No.: 29;
• A polypeptide of SEQ ID No.: 30;
• A polypeptide of SEQ ID No.: 31; and/or
• A polypeptide of SEQ ID No.: 32;
as determined for human or animal individuals not suffering from ongoing or imminent CRC development

15. Use of diagnostic markers of any of claims 1 or 2 and/or contrast agents of any of claims 5 to 8 for differentiating progressive (high-risk) CRC (adenocarcinomas) from non-progressive (low-risk) colorectal adenomas.


SEQUENCE LISTING

<110>  Philips

<120>  Protein-based methods and compositions for
the diagnosis of
       colorectal adenocarcinoma

<130>  PH012911EP1

<160>  32

<170>  PatentIn version 3.3

<210>  1
<211>  1896
<212>  PRT
<213>  Homo sapiens

<400>  1

<210>  2
<211>  541
<212>  PRT
<213>  Homo sapiens

<400>  2

Met Val Ala Asp Pro Pro Arg Asp Ser Lys Gly Leu Ala
Ala Ala Glu
1               5                   10
    15

Pro Thr Ala Asn Gly Gly Leu Ala Leu Ala Ser Ile Glu
Asp Gln Gly
            20                  25
30

Ala Ala Ala Gly Gly Tyr Cys Gly Ser Arg Asp Gln Val
Arg Arg Cys
        35                  40                  45

Leu Arg Ala Asn Leu Leu Val Leu Leu Thr Val Val Ala
Val Val Ala
    50                  55                  60

Gly Val Ala Leu Gly Leu Gly Val Ser Gly Ala Gly Gly
Ala Leu Ala
65                  70                  75
        80

Leu Gly Pro Glu Arg Leu Ser Ala Phe Val Phe Pro Gly
Glu Leu Leu
                85                  90
    95

Leu Arg Leu Leu Arg Met Ile Ile Leu Pro Leu Val Val
Cys Ser Leu
            100                 105
110

Ile Gly Gly Ala Ala Ser Leu Asp Pro Gly Ala Leu Gly
Arg Leu Gly
        115                 120                 125

Ala Trp Ala Leu Leu Phe Phe Leu Val Thr Thr Leu Leu
Ala Ser Ala
    130                 135                 140

Leu Gly Val Gly Leu Ala Leu Ala Leu Gln Pro Gly Ala
Ala Ser Ala
145                 150                 155
        160

Ala Ile Asn Ala Ser Val Gly Ala Ala Gly Ser Ala Glu
Asn Ala Pro
                165                 170
    175

Ser Lys Glu Val Leu Asp Ser Phe Leu Asp Leu Ala Arg
Asn Ile Phe
            180                     185
190

Pro Ser Asn Leu Val Ser Ala Ala Phe Arg Ser Tyr Ser
Thr Thr Tyr
        195                     200                     205

Glu Glu Arg Asn Ile Thr Gly Thr Arg Val Lys Val Pro
Val Gly Gln
    210                     215                     220

Glu Val Glu Gly Met Asn Ile Leu Gly Leu Val Val Phe
Ala Ile Val
225                     230                     235
        240

Phe Gly Val Ala Leu Arg Lys Leu Gly Pro Glu Gly Glu
Leu Leu Ile
                245                     250
    255

Arg Phe Phe Asn Ser Phe Asn Glu Ala Thr Met Val Leu
Val Ser Trp
            260                     265
270

Ile Met Trp Tyr Ala Pro Val Gly Ile Met Phe Leu Val
Ala Gly Lys
        275                     280                     285

Ile Val Glu Met Glu Asp Val Gly Leu Leu Phe Ala Arg
Leu Gly Lys

290                 295                 300

Tyr Ile Leu Cys Cys Leu Leu Gly His Ala Ile His Gly
Leu Leu Val
305                 310                 315
        320

Leu Pro Leu Ile Tyr Phe Leu Phe Thr Arg Lys Asn Pro
Tyr Arg Phe
                325                 330
    335

Leu Trp Gly Ile Val Thr Pro Leu Ala Thr Ala Phe Gly
Thr Ser Ser
            340                 345
350

Ser Ser Ala Thr Leu Pro Leu Met Met Lys Cys Val Glu
Glu Asn Asn
        355                 360                 365

Gly Val Ala Lys His Ile Ser Arg Phe Ile Leu Pro Ile
Gly Ala Thr
    370                 375                 380

Val Asn Met Asp Gly Ala Ala Leu Phe Gln Cys Val Ala
Ala Val Phe
385                 390                 395
        400

Ile Ala Gln Leu Ser Gln Gln Ser Leu Asp Phe Val Lys
Ile Ile Thr
                405                 410
    415

Ile Leu Val Thr Ala Thr Ala Ser Ser Val Gly Ala Ala
Gly Ile Pro
420 425
430

Ala Gly Gly Val Leu Thr Leu Ala Ile Ile Leu Glu Ala
Val Asn Leu
435 440 445

Pro Val Asp His Ile Ser Leu Ile Leu Ala Val Asp Trp
Leu Val Asp
450 455 460

Arg Ser Cys Thr Val Leu Asn Val Glu Gly Asp Ala Leu
Gly Ala Gly
465 470 475
480

Leu Leu Gln Asn Tyr Val Asp Arg Thr Glu Ser Arg Ser
Thr Glu Pro
485 490
495

Glu Leu Ile Gln Val Lys Ser Glu Leu Pro Leu Asp Pro
Leu Pro Val
500 505
510

Pro Thr Glu Glu Gly Asn Pro Leu Leu Lys His Tyr Arg
Gly Pro Ala
515 520 525

Gly Asp Ala Thr Val Ala Ser Glu Lys Glu Ser Val Met
    530                 535                 540

<210>  3
<211>  509
<212>  PRT
<213>  Homo sapiens

<400>  3

Met Gly Cys Ser Ala Lys Ala Arg Trp Ala Ala Gly Ala
Leu Gly Val
1               5                   10
    15

Ala Gly Leu Leu Cys Ala Val Leu Gly Ala Val Met Ile
Val Met Val
            20                  25
30

Pro Ser Leu Ile Lys Gln Gln Val Leu Lys Asn Val Arg
Ile Asp Pro
        35                  40                  45

Ser Ser Leu Ser Phe Asn Met Trp Lys Glu Ile Pro Ile
Pro Phe Tyr
    50                  55                  60

Leu Ser Val Tyr Phe Phe Asp Val Met Asn Pro Ser Glu
Ile Leu Lys
65                  70                  75
        80

Gly Glu Lys Pro Gln Val Arg Glu Arg Gly Pro Tyr Val
Tyr Arg Glu
                85                  90
    95

Phe Arg His Lys Ser Asn Ile Thr Phe Asn Asn Asn Asp

Thr Val Ser
        100                 105
110

Phe Leu Glu Tyr Arg Thr Phe Gln Phe Gln Pro Ser Lys
Ser His Gly
    115                 120                 125

Ser Glu Ser Asp Tyr Ile Val Met Pro Asn Ile Leu Val
Leu Gly Ala
    130                 135                 140

Ala Val Met Met Glu Asn Lys Pro Met Thr Leu Lys Leu
Ile Met Thr
145                 150                 155
    160

Leu Ala Phe Thr Thr Leu Gly Glu Arg Ala Phe Met Asn
Arg Thr Val
            165                 170
    175

Gly Glu Ile Met Trp Gly Tyr Lys Asp Pro Leu Val Asn
Leu Ile Asn
        180                 185
190

Lys Tyr Phe Pro Gly Met Phe Pro Phe Lys Asp Lys Phe
Gly Leu Phe
    195                 200                 205

Ala Glu Leu Asn Asn Ser Asp Ser Gly Leu Phe Thr Val
Phe Thr Gly
    210                 215                 220

Val Gln Asn Ile Ser Arg Ile His Leu Val Asp Lys Trp
Asn Gly Leu
225                 230                 235
        240

Ser Lys Val Asp Phe Trp His Ser Asp Gln Cys Asn Met
Ile Asn Gly
                245                 250
    255

Thr Ser Gly Gln Met Trp Pro Pro Phe Met Thr Pro Glu
Ser Ser Leu
            260                 265
270

Glu Phe Tyr Ser Pro Glu Ala Cys Arg Ser Met Lys Leu
Met Tyr Lys
        275                 280                 285

Glu Ser Gly Val Phe Glu Gly Ile Pro Thr Tyr Arg Phe
Val Ala Pro
    290                 295                 300

Lys Thr Leu Phe Ala Asn Gly Ser Ile Tyr Pro Pro Asn
Glu Gly Phe
305                 310                 315
        320

Cys Pro Cys Leu Glu Ser Gly Ile Gln Asn Val Ser Thr
Cys Arg Phe
                325                 330
    335

Ser Ala Pro Leu Phe Leu Ser His Pro His Phe Leu Asn
Ala Asp Pro
            340                 345

350

Val Leu Ala Glu Ala Val Thr Gly Leu His Pro Asn Gln
Glu Ala His
        355                 360                 365

Ser Leu Phe Leu Asp Ile His Pro Val Thr Gly Ile Pro
Met Asn Cys
    370                 375                 380

Ser Val Lys Leu Gln Leu Ser Leu Tyr Met Lys Ser Val
Ala Gly Ile
385                 390                 395
        400

Gly Gln Thr Gly Lys Ile Glu Pro Val Val Leu Pro Leu
Leu Trp Phe
                405                 410
    415

Ala Glu Ser Gly Ala Met Glu Gly Glu Thr Leu His Thr
Phe Tyr Thr
            420                 425
430

Gln Leu Val Leu Met Pro Lys Val Met His Tyr Ala Gln
Tyr Val Leu
        435                 440                 445

Leu Ala Leu Gly Cys Val Leu Leu Leu Val Pro Val Ile
Cys Gln Ile
    450                 455                 460

Arg Ser Gln Glu Lys Cys Tyr Leu Phe Trp Ser Ser Ser

Lys Lys Gly
465                 470                 475
        480

Ser Lys Asp Lys Glu Ala Ile Gln Ala Tyr Ser Glu Ser
Leu Met Thr
                485                 490
    495

Ser Ala Pro Lys Gly Ser Val Leu Gln Glu Ala Lys Leu
            500                 505

<210>  4
<211>  532
<212>  PRT
<213>  Homo sapiens

<400>  4

Met Ala Pro Ser Ser Pro Arg Pro Ala Leu Pro Ala Leu
Leu Val Leu
1               5                   10
    15

Leu Gly Ala Leu Phe Pro Gly Pro Gly Asn Ala Gln Thr
Ser Val Ser
            20                  25
30

Pro Ser Lys Val Ile Leu Pro Arg Gly Gly Ser Val Leu
Val Thr Cys
        35                  40                  45

Ser Thr Ser Cys Asp Gln Pro Lys Leu Leu Gly Ile Glu
Thr Pro Leu
    50                  55                  60

Pro Lys Lys Glu Leu Leu Leu Pro Gly Asn Asn Arg Lys
Val Tyr Glu
65                  70                  75
        80

Leu Ser Asn Val Gln Glu Asp Ser Gln Pro Met Cys Tyr
Ser Asn Cys
                85                  90
    95

Pro Asp Gly Gln Ser Thr Ala Lys Thr Phe Leu Thr Val
Tyr Trp Thr
            100                 105
110

Pro Glu Arg Val Glu Leu Ala Pro Leu Pro Ser Trp Gln
Pro Val Gly
        115                 120                 125

Lys Asn Leu Thr Leu Arg Cys Gln Val Glu Gly Gly Ala
Pro Arg Ala
    130                 135                 140

Asn Leu Thr Val Val Leu Leu Arg Gly Glu Lys Glu Leu
Lys Arg Glu
145                 150                 155
        160

Pro Ala Val Gly Glu Pro Ala Glu Val Thr Thr Thr Val
Leu Val Arg
                165                 170
    175

Arg Asp His His Gly Ala Asn Phe Ser Cys Arg Thr Glu
Leu Asp Leu

180                     185
190

Arg Pro Gln Gly Leu Glu Leu Phe Glu Asn Thr Ser Ala
Pro Tyr Gln
        195                     200                     205

Leu Gln Thr Phe Val Leu Pro Ala Thr Pro Pro Gln Leu
Val Ser Pro
    210                     215                     220

Arg Val Leu Glu Val Asp Thr Gln Gly Thr Val Val Cys
Ser Leu Asp
225                     230                     235
        240

Gly Leu Phe Pro Val Ser Glu Ala Gln Val His Leu Ala
Leu Gly Asp
                245                     250
    255

Gln Arg Leu Asn Pro Thr Val Thr Tyr Gly Asn Asp Ser
Phe Ser Ala
            260                     265
270

Lys Ala Ser Val Ser Val Thr Ala Glu Asp Glu Gly Thr
Gln Arg Leu
        275                     280                     285

Thr Cys Ala Val Ile Leu Gly Asn Gln Ser Gln Glu Thr
Leu Gln Thr
    290                     295                     300

Val Thr Ile Tyr Ser Phe Pro Ala Pro Asn Val Ile Leu
Thr Lys Pro
305                 310                 315
        320

Glu Val Ser Glu Gly Thr Glu Val Thr Val Lys Cys Glu
Ala His Pro
                325                 330
    335

Arg Ala Lys Val Thr Leu Asn Gly Val Pro Ala Gln Pro
Leu Gly Pro
            340                 345
350

Arg Ala Gln Leu Leu Leu Lys Ala Thr Pro Glu Asp Asn
Gly Arg Ser
        355                 360                 365

Phe Ser Cys Ser Ala Thr Leu Glu Val Ala Gly Gln Leu
Ile His Lys
    370                 375                 380

Asn Gln Thr Arg Glu Leu Arg Val Leu Tyr Gly Pro Arg
Leu Asp Glu
385                 390                 395
        400

Arg Asp Cys Pro Gly Asn Trp Thr Trp Pro Glu Asn Ser
Gln Gln Thr
                405                 410
    415

Pro Met Cys Gln Ala Trp Gly Asn Pro Leu Pro Glu Leu
Lys Cys Leu
            420                 425
430

Lys Asp Gly Thr Phe Pro Leu Pro Ile Gly Glu Ser Val
Thr Val Thr
        435                 440                 445

Arg Asp Leu Glu Gly Thr Tyr Leu Cys Arg Ala Arg Ser
Thr Gln Gly
    450                 455                 460

Glu Val Thr Arg Lys Val Thr Val Asn Val Leu Ser Pro
Arg Tyr Glu
465                 470                 475
        480

Ile Val Ile Ile Thr Val Val Ala Ala Ala Val Ile Met
Gly Thr Ala
                485                 490
    495

Gly Leu Ser Thr Tyr Leu Tyr Asn Arg Gln Arg Lys Ile
Lys Lys Tyr
            500                 505
510

Arg Leu Gln Gln Ala Gln Lys Gly Thr Pro Met Lys Pro
Asn Thr Gln
        515                 520                 525

Ala Thr Pro Pro
    530

<210>  5
<211>  628
<212>  PRT
<213>  Homo sapiens

<400>  5

Met Glu Pro Pro Asp Ala Pro Ala Gln Ala Arg Gly Ala
Pro Arg Leu
1               5                   10
    15

Leu Leu Leu Ala Val Leu Leu Ala Ala His Pro Asp Ala
Gln Ala Glu
            20                  25
30

Val Arg Leu Ser Val Pro Pro Leu Val Glu Val Met Arg
Gly Lys Ser
        35                  40                  45

Val Ile Leu Asp Cys Thr Pro Thr Gly Thr His Asp His
Tyr Met Leu
    50                  55                  60

Glu Trp Phe Leu Thr Asp Arg Ser Gly Ala Arg Pro Arg
Leu Ala Ser
65                  70                  75
        80

Ala Glu Met Gln Gly Ser Glu Leu Gln Val Thr Met His
Asp Thr Arg
                85                  90
    95

Gly Arg Ser Pro Pro Tyr Gln Leu Asp Ser Gln Gly Arg
Leu Val Leu
            100                 105
110

Ala Glu Ala Gln Val Gly Asp Glu Arg Asp Tyr Val Cys

Val Val Arg
        115                 120                 125

Ala Gly Ala Ala Gly Thr Ala Glu Ala Thr Ala Arg Leu
Asn Val Phe
    130                 135                 140

Ala Lys Pro Glu Ala Thr Glu Val Ser Pro Asn Lys Gly
Thr Leu Ser
145                 150                 155
        160

Val Met Glu Asp Ser Ala Gln Glu Ile Ala Thr Cys Asn
Ser Arg Asn
                165                 170
    175

Gly Asn Pro Ala Pro Lys Ile Thr Trp Tyr Arg Asn Gly
Gln Arg Leu
            180                 185
190

Glu Val Pro Val Glu Met Asn Pro Glu Gly Tyr Met Thr
Ser Arg Thr
        195                 200                 205

Val Arg Glu Ala Ser Gly Leu Leu Ser Leu Thr Ser Thr
Leu Tyr Leu
    210                 215                 220

Arg Leu Arg Lys Asp Asp Arg Asp Ala Ser Phe His Cys
Ala Ala His
225                 230                 235
        240

Tyr Ser Leu Pro Glu Gly Arg His Gly Arg Leu Asp Ser
Pro Thr Phe
                245                     250
    255

His Leu Thr Leu His Tyr Pro Thr Glu His Val Gln Phe
Trp Val Gly
            260                     265
270

Ser Pro Ser Thr Pro Ala Gly Trp Val Arg Glu Gly Asp
Thr Val Gln
        275                     280                     285

Leu Leu Cys Arg Gly Asp Gly Ser Pro Ser Pro Glu Tyr
Thr Leu Phe
    290                     295                     300

Arg Leu Gln Asp Glu Gln Glu Glu Val Leu Asn Val Asn
Leu Glu Gly
305                     310                     315
        320

Asn Leu Thr Leu Glu Gly Val Thr Arg Gly Gln Ser Gly
Thr Tyr Gly
                325                     330
    335

Cys Arg Val Glu Asp Tyr Asp Ala Ala Asp Asp Val Gln
Leu Ser Lys
            340                     345
350

Thr Leu Glu Leu Arg Val Ala Tyr Leu Asp Pro Leu Glu
Leu Ser Glu
        355                     360                     365

Gly Lys Val Leu Ser Leu Pro Leu Asn Ser Ser Ala Val
Val Asn Cys
    370                 375                 380

Ser Val His Gly Leu Pro Thr Pro Ala Leu Arg Trp Thr
Lys Asp Ser
385                 390                 395
        400

Thr Pro Leu Gly Asp Gly Pro Met Leu Ser Leu Ser Ser
Ile Thr Phe
                405                 410
    415

Asp Ser Asn Gly Thr Tyr Val Cys Glu Ala Ser Leu Pro
Thr Val Pro
            420                 425
430

Val Leu Ser Arg Thr Gln Asn Phe Thr Leu Leu Val Gln
Gly Ser Pro
        435                 440                 445

Glu Leu Lys Thr Ala Glu Ile Glu Pro Lys Ala Asp Gly
Ser Trp Arg
    450                 455                 460

Glu Gly Asp Glu Val Thr Leu Ile Cys Ser Ala Arg Gly
His Pro Asp
465                 470                 475
        480

Pro Lys Leu Ser Trp Ser Gln Leu Gly Gly Ser Pro Ala

Glu Pro Ile
                485                 490
    495

Pro Gly Arg Gln Gly Trp Val Ser Ser Ser Leu Thr Leu
Lys Val Thr
            500                 505
510

Ser Ala Leu Ser Arg Asp Gly Ile Ser Cys Glu Ala Ser
Asn Pro His
        515                 520                 525

Gly Asn Lys Arg His Val Phe His Phe Gly Thr Val Ser
Pro Gln Thr
    530                 535                 540

Ser Gln Ala Gly Val Ala Val Met Ala Val Ala Val Ser
Val Gly Leu
545                 550                 555
        560

Leu Leu Leu Val Val Ala Val Phe Tyr Cys Val Arg Arg
Lys Gly Gly
                565                 570
    575

Pro Cys Cys Arg Gln Arg Arg Glu Lys Gly Ala Pro Pro
Pro Gly Glu
            580                 585
590

Pro Gly Leu Ser His Ser Gly Ser Glu Gln Pro Glu Gln
Thr Gly Leu
        595                 600                 605

Leu Met Gly Gly Ala Ser Gly Gly Ala Arg Gly Gly Ser
Gly Gly Phe
    610                 615                 620

Gly Asp Glu Cys
625

<210>  6
<211>  253
<212>  PRT
<213>  Homo sapiens

<400>  6

<210>  7
<211>  895
<212>  PRT
<213>  Homo sapiens

<400>  7

Met Arg Met Ser Val Gly Leu Ser Leu Leu Leu Pro Leu
Ser Gly Arg
1               5                   10
    15

Thr Phe Leu Leu Leu Leu Ser Val Val Met Ala Gln Ser
His Trp Pro
            20                  25
30

Ser Glu Pro Ser Glu Ala Val Arg Asp Trp Glu Asn Gln
Leu Glu Ala
        35                  40                  45

Ser Met His Ser Val Leu Ser Asp Leu His Glu Ala Val
Pro Thr Val
    50                  55                  60

Val Gly Ile Pro Asp Gly Thr Ala Val Val Gly Arg Ser
Phe Arg Val
65                  70                  75
        80

Thr Ile Pro Thr Asp Leu Ile Ala Ser Ser Gly Asp Ile
Ile Lys Val
                85                  90
    95

Ser Ala Ala Gly Lys Glu Ala Leu Pro Ser Trp Leu His
Trp Asp Ser
            100                 105
110

Gln Ser His Thr Leu Glu Gly Leu Pro Leu Asp Thr Asp
Lys Gly Val
        115                 120                 125

His Tyr Ile Ser Val Ser Ala Thr Arg Leu Gly Ala Asn
Gly Ser His
    130                 135                 140

Ile Pro Gln Thr Ser Ser Val Phe Ser Ile Glu Val Tyr
Pro Glu Asp
145                 150                 155
        160

His Ser Glu Leu Gln Ser Val Arg Thr Ala Ser Pro Asp
Pro Gly Glu
                165                 170

175

Val Val Ser Ser Ala Cys Ala Ala Asp Glu Pro Val Thr
Val Leu Thr
                180                 185
190

Val Ile Leu Asp Ala Asp Leu Thr Lys Met Thr Pro Lys
Gln Arg Ile
        195                 200                 205

Asp Leu Leu His Arg Met Arg Ser Phe Ser Glu Val Glu
Leu His Asn
    210                 215                 220

Met Lys Leu Val Pro Val Val Asn Asn Arg Leu Phe Asp
Met Ser Ala
225                 230                 235
        240

Phe Met Ala Gly Pro Gly Asn Ala Lys Lys Val Val Glu
Asn Gly Ala
                245                 250
    255

Leu Leu Ser Trp Lys Leu Gly Cys Ser Leu Asn Gln Asn
Ser Val Pro
                260                 265
270

Asp Ile His Gly Val Glu Ala Pro Ala Arg Glu Gly Ala
Met Ser Ala
        275                 280                 285

Gln Leu Gly Tyr Pro Val Val Gly Trp His Ile Ala Asn

Lys Lys Pro
    290                 295                 300

Pro Leu Pro Lys Arg Val Arg Arg Gln Ile His Ala Thr
Pro Thr Pro
305                 310                 315
        320

Val Thr Ala Ile Gly Pro Pro Thr Thr Ala Ile Gln Glu
Pro Pro Ser
                325                 330
    335

Arg Ile Val Pro Thr Pro Thr Ser Pro Ala Ile Ala Pro
Pro Thr Glu
            340                 345
350

Thr Met Ala Pro Pro Val Arg Asp Pro Val Pro Gly Lys
Pro Thr Val
        355                 360                 365

Thr Ile Arg Thr Arg Gly Ala Ile Ile Gln Thr Pro Thr
Leu Gly Pro
    370                 375                 380

Ile Gln Pro Thr Arg Val Ser Glu Ala Gly Thr Thr Val
Pro Gly Gln
385                 390                 395
        400

Ile Arg Pro Thr Met Thr Ile Pro Gly Tyr Val Glu Pro
Thr Ala Val
                405                 410
    415

Ala Thr Pro Pro Thr Thr Thr Thr Lys Lys Pro Arg Val
Ser Thr Pro
            420                     425
430

Lys Pro Ala Thr Pro Ser Thr Asp Ser Thr Thr Thr Thr
Thr Arg Arg
        435                     440                     445

Pro Thr Lys Lys Pro Arg Thr Pro Arg Pro Val Pro Arg
Val Thr Thr
    450                     455                     460

Lys Val Ser Ile Thr Arg Leu Glu Thr Ala Ser Pro Pro
Thr Arg Ile
465                     470                     475
        480

Arg Thr Thr Thr Ser Gly Val Pro Arg Gly Gly Glu Pro
Asn Gln Arg
                485                     490
    495

Pro Glu Leu Lys Asn His Ile Asp Arg Val Asp Ala Trp
Val Gly Thr
            500                     505
510

Tyr Phe Glu Val Lys Ile Pro Ser Asp Thr Phe Tyr Asp
His Glu Asp
        515                     520                     525

Thr Thr Thr Asp Lys Leu Lys Leu Thr Leu Lys Leu Arg
Glu Gln Gln
    530                     535                     540

Leu Val Gly Glu Lys Ser Trp Val Gln Phe Asn Ser Asn
Ser Gln Leu
545                 550                 555
      560

Met Tyr Gly Leu Pro Asp Ser Ser His Val Gly Lys His
Glu Tyr Phe
            565                 570
    575

Met His Ala Thr Asp Lys Gly Gly Leu Ser Ala Val Asp
Ala Phe Glu
        580                 585
590

Ile His Val His Arg Arg Pro Gln Gly Asp Arg Ala Pro
Ala Arg Phe
      595                 600                 605

Lys Ala Lys Phe Val Gly Asp Pro Ala Leu Val Leu Asn
Asp Ile His
    610                 615                 620

Lys Lys Ile Ala Leu Val Lys Lys Leu Ala Phe Ala Phe
Gly Asp Arg
625                 630                 635
      640

Asn Cys Ser Thr Ile Thr Leu Gln Asn Ile Thr Arg Gly
Ser Ile Val
            645                 650
    655

Val Glu Trp Thr Asn Asn Thr Leu Pro Leu Glu Pro Cys

Pro Lys Glu
            660                 665
670

Gln Ile Ala Gly Leu Ser Arg Arg Ile Ala Glu Asp Asp
Gly Lys Pro
        675                 680                 685

Arg Pro Ala Phe Ser Asn Ala Leu Glu Pro Asp Phe Lys
Ala Thr Ser
    690                 695                 700

Ile Thr Val Thr Gly Ser Gly Ser Cys Arg His Leu Gln
Phe Ile Pro
705                 710                 715
        720

Val Val Pro Pro Arg Arg Val Pro Ser Glu Ala Pro Pro
Thr Glu Val
                725                 730
    735

Pro Asp Arg Asp Pro Glu Lys Ser Ser Glu Asp Asp Val
Tyr Leu His
            740                 745
750

Thr Val Ile Pro Ala Val Val Val Ala Ala Ile Leu Leu
Ile Ala Gly
        755                 760                 765

Ile Ile Ala Met Ile Cys Tyr Arg Lys Lys Arg Lys Gly
Lys Leu Thr
    770                 775                 780

Leu Glu Asp Gln Ala Thr Phe Ile Lys Lys Gly Val Pro
Ile Ile Phe
785                 790                 795
        800

Ala Asp Glu Leu Asp Asp Ser Lys Pro Pro Pro Ser Ser
Ser Met Pro
                805                 810
    815

Leu Ile Leu Gln Glu Glu Lys Ala Pro Leu Pro Pro Pro
Glu Tyr Pro
            820                 825
830

Asn Gln Ser Val Pro Glu Thr Thr Pro Leu Asn Gln Asp
Thr Met Gly
        835                 840                 845

Glu Tyr Thr Pro Leu Arg Asp Glu Asp Pro Asn Ala Pro
Pro Tyr Gln
    850                 855                 860

Pro Pro Pro Pro Phe Thr Ala Pro Met Glu Gly Lys Gly
Ser Arg Pro
865                 870                 875
        880

Lys Asn Met Thr Pro Tyr Arg Ser Pro Pro Pro Tyr Val
Pro Pro
                885                 890
    895

<210>  8
<211>  926
<212>  PRT

<213>  Homo sapiens

<400>  8

Ser Ala Arg Glu Glu Ser Gly Ala Gly Ala Arg Pro Arg
Arg Arg Ser
1               5                   10
    15

Ala Asp Ser Gly Ala Ala Gly Ala Gly Arg Gly Gly Gly
Gly Glu Ala
            20                  25
30

Ala Gly Lys Glu Glu Glu Gly Glu Ser Arg Ser Arg Arg
Ala Ser Met
        35                  40                  45

Gly Arg Leu Ala Ser Arg Pro Leu Leu Leu Ala Leu Leu
Ser Leu Ala
    50                  55                  60

Leu Cys Arg Gly Arg Val Val Arg Val Pro Thr Ala Thr
Leu Val Arg
65                  70                  75
        80

Val Val Gly Thr Glu Leu Val Ile Pro Cys Asn Val Ser
Asp Tyr Asp
                85                  90
    95

Gly Pro Ser Glu Gln Asn Phe Asp Trp Ser Phe Ser Ser
Leu Gly Ser
            100                 105
110

Ser Phe Val Glu Leu Ala Ser Thr Trp Glu Val Gly Phe
Pro Ala Gln
        115                 120                 125

Leu Tyr Gln Glu Arg Leu Gln Arg Gly Glu Ile Leu Leu
Arg Arg Thr
    130                 135                 140

Ala Asn Asp Ala Val Glu Leu His Ile Lys Asn Val Gln
Pro Ser Asp
145                 150                 155
        160

Gln Gly His Tyr Lys Cys Ser Thr Pro Ser Thr Asp Ala
Thr Val Gln
                165                 170
    175

Gly Asn Tyr Glu Asp Thr Val Gln Val Lys Val Leu Ala
Asp Ser Leu
            180                 185
190

His Val Gly Pro Ser Ala Arg Pro Pro Pro Ser Leu Ser
Leu Arg Glu
        195                 200                 205

Gly Glu Pro Phe Glu Leu Arg Cys Thr Ala Ala Ser Ala
Ser Pro Leu
    210                 215                 220

His Thr His Leu Ala Leu Leu Trp Glu Val His Arg Gly
Pro Ala Arg
225                 230                 235
        240

Arg Ser Val Leu Ala Leu Thr His Glu Gly Arg Phe His
Pro Gly Leu
                245                     250
    255

Gly Tyr Glu Gln Arg Tyr His Ser Gly Asp Val Arg Leu
Asp Thr Val
            260                     265
270

Gly Ser Asp Ala Tyr Arg Leu Ser Val Ser Arg Ala Leu
Ser Ala Asp
        275                     280                     285

Gln Gly Ser Tyr Arg Cys Ile Val Ser Glu Trp Ile Ala
Glu Gln Gly
    290                     295                     300

Asn Trp Gln Glu Ile Gln Glu Lys Ala Val Glu Val Ala
Thr Val Val
305                     310                     315
        320

Ile Gln Pro Ser Val Leu Arg Ala Ala Val Pro Lys Asn
Val Ser Val
                325                     330
    335

Ala Glu Gly Lys Glu Leu Asp Leu Thr Cys Asn Ile Thr
Thr Asp Arg
            340                     345
350

Ala Asp Asp Val Arg Pro Glu Val Thr Trp Ser Phe Ser
Arg Met Pro

355                 360                 365

Asp Ser Thr Leu Pro Gly Ser Arg Val Leu Ala Arg Leu
Asp Arg Asp
    370                 375                 380

Ser Leu Val His Ser Ser Pro His Val Ala Leu Ser His
Val Asp Ala
385                 390                 395
        400

Arg Ser Tyr His Leu Leu Val Arg Asp Val Ser Lys Glu
Asn Ser Gly
                405                 410
    415

Tyr Tyr Tyr Cys His Val Ser Leu Trp Ala Pro Gly His
Asn Arg Ser
            420                 425
430

Trp His Lys Val Ala Glu Ala Val Ser Ser Pro Ala Gly
Val Gly Val
        435                 440                 445

Thr Trp Leu Glu Pro Asp Tyr Gln Val Tyr Leu Asn Ala
Ser Lys Val
    450                 455                 460

Pro Gly Phe Ala Asp Asp Pro Thr Glu Leu Ala Cys Arg
Val Val Asp
465                 470                 475
        480

Thr Lys Ser Gly Glu Ala Asn Val Arg Phe Thr Val Ser
Trp Tyr Tyr
                485                     490
    495

Arg Met Asn Arg Arg Ser Asp Asn Val Val Thr Ser Glu
Leu Leu Ala
            500                     505
510

Val Met Asp Gly Asp Trp Thr Leu Lys Tyr Gly Glu Arg
Ser Lys Gln
        515                     520                     525

Arg Ala Gln Asp Gly Asp Phe Ile Phe Ser Lys Glu His
Thr Asp Thr
    530                     535                     540

Phe Asn Phe Arg Ile Gln Arg Thr Thr Glu Glu Asp Arg
Gly Asn Tyr
545                     550                     555
        560

Tyr Cys Val Val Ser Ala Trp Thr Lys Gln Arg Asn Asn
Ser Trp Val
                565                     570
    575

Lys Ser Lys Asp Val Phe Ser Lys Pro Val Asn Ile Phe
Trp Ala Leu
            580                     585
590

Glu Asp Ser Val Leu Val Val Lys Ala Arg Gln Pro Lys
Pro Phe Phe
        595                     600                     605

Ala Ala Gly Asn Thr Phe Glu Met Thr Cys Lys Val Ser
Ser Lys Asn
    610                 615                 620

Ile Lys Ser Pro Arg Tyr Ser Val Leu Ile Met Ala Glu
Lys Pro Val
625                 630                 635
        640

Gly Asp Leu Ser Ser Pro Asn Glu Thr Lys Tyr Ile Ile
Ser Leu Asp
                645                 650
    655

Gln Asp Ser Val Val Lys Leu Glu Asn Trp Thr Asp Ala
Ser Arg Val
            660                 665
670

Asp Gly Val Val Leu Glu Lys Val Gln Glu Asp Glu Phe
Arg Tyr Arg
        675                 680                 685

Met Tyr Gln Thr Gln Val Ser Asp Ala Gly Leu Tyr Arg
Cys Met Val
    690                 695                 700

Thr Ala Trp Ser Pro Val Arg Gly Ser Leu Trp Arg Glu
Ala Ala Thr
705                 710                 715
        720

Ser Leu Ser Asn Pro Ile Glu Ile Asp Phe Gln Thr Ser
Gly Pro Ile

725                    730
735

Phe Asn Ala Ser Val His Ser Asp Thr Pro Ser Val Ile
Arg Gly Asp
            740                 745
750

Leu Ile Lys Leu Phe Cys Ile Ile Thr Val Glu Gly Ala
Ala Leu Asp
        755                 760                 765

Pro Asp Asp Met Ala Phe Asp Val Ser Trp Phe Ala Val
His Ser Phe
    770                 775                 780

Gly Leu Asp Lys Ala Pro Val Leu Leu Ser Ser Leu Asp
Arg Lys Gly
785                 790                 795
        800

Ile Val Thr Thr Ser Arg Arg Asp Trp Lys Ser Asp Leu
Ser Leu Glu
                805                 810
    815

Arg Val Ser Val Leu Glu Phe Leu Leu Gln Val His Gly
Ser Glu Asp
            820                 825
830

Gln Asp Phe Gly Asn Tyr Tyr Cys Ser Val Thr Pro Trp
Val Lys Ser
        835                 840                 845

Pro Thr Gly Ser Trp Gln Lys Glu Ala Glu Ile His Ser
Lys Pro Val
    850                 855                 860

Phe Ile Thr Val Lys Met Asp Val Leu Asn Ala Phe Lys
Tyr Pro Leu
865                 870                 875
        880

Leu Ile Gly Val Gly Leu Ser Thr Val Ile Gly Leu Leu
Ser Cys Leu
                885                 890
    895

Ile Gly Tyr Cys Ser Ser His Trp Cys Cys Lys Lys Glu
Val Gln Glu
            900                 905
910

Thr Arg Arg Glu Arg Arg Arg Leu Met Ser Met Glu Met
Asp
        915                 920                 925

<210>  9
<211>  613
<212>  PRT
<213>  Homo sapiens

<400>  9

Met Gly Ala Leu Arg Pro Thr Leu Leu Pro Pro Ser Leu
Pro Leu Leu
1               5                   10
    15

Leu Leu Leu Met Leu Gly Met Gly Cys Trp Ala Arg Glu
Val Leu Val
            20                  25

30

Pro Glu Gly Pro Leu Tyr Arg Val Ala Gly Thr Ala Val
Ser Ile Ser
        35                  40                  45

Cys Asn Val Thr Gly Tyr Glu Gly Pro Ala Gln Gln Asn
Phe Glu Trp
    50                  55                  60

Phe Leu Tyr Arg Pro Glu Ala Pro Asp Thr Ala Leu Gly
Ile Val Ser
65                  70                  75
        80

Thr Lys Asp Thr Gln Phe Ser Tyr Ala Val Phe Lys Ser
Arg Val Val
                85                  90
    95

Ala Gly Glu Val Gln Val Gln Arg Leu Gln Gly Asp Ala
Val Val Leu
            100                 105
110

Lys Ile Ala Arg Leu Gln Ala Gln Asp Ala Gly Ile Tyr
Glu Cys His
        115                 120                 125

Thr Pro Ser Thr Asp Thr Arg Tyr Leu Gly Ser Tyr Ser
Gly Lys Val
    130                 135                 140

Glu Leu Arg Val Leu Pro Asp Val Leu Gln Val Ser Ala

Ala Pro Pro
145                 150                 155
    160

Gly Pro Arg Gly Arg Gln Ala Pro Thr Ser Pro Pro Arg
Met Thr Val
                165                 170
    175

His Glu Gly Gln Glu Leu Ala Leu Gly Cys Leu Ala Arg
Thr Ser Thr
            180                 185
190

Gln Lys His Thr His Leu Ala Val Ser Phe Gly Arg Ser
Val Pro Glu
        195                 200                 205

Ala Pro Val Gly Arg Ser Thr Leu Gln Glu Val Val Gly
Ile Arg Ser
    210                 215                 220

Asp Leu Ala Val Glu Ala Gly Ala Pro Tyr Ala Glu Arg
Leu Ala Ala
225                 230                 235
    240

Gly Glu Leu Arg Leu Gly Lys Glu Gly Thr Asp Arg Tyr
Arg Met Val
                245                 250
    255

Val Gly Gly Ala Gln Ala Gly Asp Ala Gly Thr Tyr His
Cys Thr Ala
            260                 265
270

Ala Glu Trp Ile Gln Asp Pro Asp Gly Ser Trp Ala Gln
Ile Ala Glu
275                 280                 285

Lys Arg Ala Val Leu Ala His Val Asp Val Gln Thr Leu
Ser Ser Gln
    290                 295                 300

Leu Ala Val Thr Val Gly Pro Gly Glu Arg Arg Ile Gly
Pro Gly Glu
305                 310                 315
        320

Pro Leu Glu Leu Leu Cys Asn Val Ser Gly Ala Leu Pro
Pro Ala Gly
                325                 330
    335

Arg His Ala Ala Tyr Ser Val Gly Trp Glu Met Ala Pro
Ala Gly Ala
            340                 345
350

Pro Gly Pro Gly Arg Leu Val Ala Gln Leu Asp Thr Glu
Gly Val Gly
        355                 360                 365

Ser Leu Gly Pro Gly Tyr Glu Gly Arg His Ile Ala Met
Glu Lys Val
    370                 375                 380

Ala Ser Arg Thr Tyr Arg Leu Arg Leu Glu Ala Ala Arg
Pro Gly Asp
385                 390                 395

400

Ala Gly Thr Tyr Arg Cys Leu Ala Lys Ala Tyr Val Arg
Gly Ser Gly
405 410
415

Thr Arg Leu Arg Glu Ala Ala Ser Ala Arg Ser Arg Pro
Leu Pro Val
420 425
430

His Val Arg Glu Glu Gly Val Val Leu Glu Ala Val Ala
Trp Leu Ala
435 440 445

Gly Gly Thr Val Tyr Arg Gly Glu Thr Ala Ser Leu Leu
Cys Asn Ile
450 455 460

Ser Val Arg Gly Gly Pro Pro Gly Leu Arg Leu Ala Ala
Ser Trp Trp
465 470 475
480

Val Glu Arg Pro Glu Asp Gly Glu Leu Ser Ser Val Pro
Ala Gln Leu
485 490
495

Val Gly Gly Val Gly Gln Asp Gly Val Ala Glu Leu Gly
Val Arg Pro
500 505
510

Gly Gly Gly Pro Val Ser Val Glu Leu Val Gly Pro Arg

Ser His Arg
        515                 520                 525

Leu Arg Leu His Ser Leu Gly Pro Glu Asp Glu Gly Val Tyr His Cys
    530                 535                 540

Ala Pro Ser Ala Trp Val Gln His Ala Asp Tyr Ser Trp Tyr Gln Ala
545                 550                 555                 560

Gly Ser Ala Arg Ser Gly Pro Val Thr Val Tyr Pro Tyr Met His Ala
                565                 570                 575

Leu Asp Thr Leu Phe Val Pro Leu Leu Val Gly Thr Gly Val Ala Leu
            580                 585                 590

Val Thr Gly Ala Thr Val Leu Gly Thr Ile Thr Cys Cys Phe Met Lys
        595                 600                 605

Arg Leu Arg Lys Arg
    610

<210>  10
<211>  860
<212>  PRT
<213>  Homo sapiens

<400>  10

<210>  11
<211>  542
<212>  PRT
<213>  Homo sapiens

<400>  11

Met Thr Lys Ser Asn Gly Glu Glu Pro Lys Met Gly Gly
Arg Met Glu
1               5                   10
    15

Arg Phe Gln Gln Gly Val Arg Lys Arg Thr Leu Leu Ala
Lys Lys Lys
            20                  25
30

Val Gln Asn Ile Thr Lys Glu Asp Val Lys Ser Tyr Leu
Phe Arg Asn
        35                  40                  45

Ala Phe Val Leu Leu Thr Val Thr Ala Val Ile Val Gly
Thr Ile Leu
    50                  55                  60

Gly Phe Thr Leu Arg Pro Tyr Arg Met Ser Tyr Arg Glu
Val Lys Tyr
65                  70                  75
        80

Phe Ser Phe Pro Gly Glu Leu Leu Met Arg Met Leu Gln
Met Leu Val
                85                  90
    95

Leu Pro Leu Ile Ile Ser Ser Leu Val Thr Gly Met Ala
Ala Leu Asp
            100                 105

110

Ser Lys Ala Ser Gly Lys Met Gly Met Arg Ala Val Val
Tyr Tyr Met
        115                 120                 125

Thr Thr Thr Ile Ile Ala Val Val Ile Gly Ile Ile Ile
Val Ile Ile
    130                 135                 140

Ile His Pro Gly Lys Gly Thr Lys Glu Asn Met His Arg
Glu Gly Lys
145                 150                 155
        160

Ile Val Arg Val Thr Ala Ala Asp Ala Phe Leu Asp Leu
Ile Arg Asn
                165                 170
    175

Met Phe Pro Pro Asn Leu Val Glu Ala Cys Phe Lys Gln
Phe Lys Thr
            180                 185
190

Asn Tyr Glu Lys Arg Ser Phe Lys Val Pro Ile Gln Ala
Asn Glu Thr
        195                 200                 205

Leu Val Gly Ala Val Ile Asn Asn Val Ser Glu Ala Met
Glu Thr Leu
    210                 215                 220

Thr Arg Ile Thr Glu Glu Leu Val Pro Val Pro Gly Ser

Val Asn Gly
225                 230                 235
        240

Val Asn Ala Leu Gly Leu Val Val Phe Ser Met Cys Phe
Gly Phe Val
                245                 250
    255

Ile Gly Asn Met Lys Glu Gln Gly Gln Ala Leu Arg Glu
Phe Phe Asp
            260                 265
270

Ser Leu Asn Glu Ala Ile Met Arg Leu Val Ala Val Ile
Met Trp Tyr
        275                 280                 285

Ala Pro Val Gly Ile Leu Phe Leu Ile Ala Gly Lys Ile
Val Glu Met
    290                 295                 300

Glu Asp Met Gly Val Ile Gly Gly Gln Leu Ala Met Tyr
Thr Val Thr
305                 310                 315
        320

Val Ile Val Gly Leu Leu Ile His Ala Val Ile Val Leu
Pro Leu Leu
                325                 330
    335

Tyr Phe Leu Val Thr Arg Lys Asn Pro Trp Val Phe Ile
Gly Gly Leu
            340                 345
350

Leu Gln Ala Leu Ile Thr Ala Leu Gly Thr Ser Ser Ser
Ser Ala Thr
        355                 360                 365

Leu Pro Ile Thr Phe Lys Cys Leu Glu Glu Asn Asn Gly
Val Asp Lys
    370                 375                 380

Arg Val Thr Arg Phe Val Leu Pro Val Gly Ala Thr Ile
Asn Met Asp
385                 390                 395
        400

Gly Thr Ala Leu Tyr Glu Ala Leu Ala Ala Ile Phe Ile
Ala Gln Val
                405                 410
    415

Asn Asn Phe Glu Leu Asn Phe Gly Gln Ile Ile Thr Ile
Ser Ile Thr
            420                 425
430

Ala Thr Ala Ala Ser Ile Gly Ala Ala Gly Ile Pro Gln
Ala Gly Leu
        435                 440                 445

Val Thr Met Val Ile Val Leu Thr Ser Val Gly Leu Pro
Thr Asp Asp
    450                 455                 460

Ile Thr Leu Ile Ile Ala Val Asp Trp Phe Leu Asp Arg
Leu Arg Thr
465                 470                 475

480

Thr Thr Asn Val Leu Gly Asp Ser Leu Gly Ala Gly Ile
Val Glu His
485 490
495

Leu Ser Arg His Glu Leu Lys Asn Arg Asp Val Glu Met
Gly Asn Ser
500 505
510

Val Ile Glu Glu Asn Glu Met Lys Lys Pro Tyr Gln Leu
Ile Ala Gln
515 520 525

Asp Asn Glu Thr Glu Lys Pro Ile Asp Ser Glu Thr Lys
Met
530 535 540

<210> 12
<211> 698
<212> PRT
<213> Homo sapiens

<400> 12

Met Gln Ala His Glu Leu Phe Arg Tyr Phe Arg Met Pro
Glu Leu Val
1 5 10
15

Asp Phe Arg Gln Tyr Val Arg Thr Leu Pro Thr Asn Thr
Leu Met Gly
20 25
30

Phe Gly Ala Phe Ala Ala Leu Thr Thr Phe Trp Tyr Ala
Thr Arg Pro
        35                  40                  45

Lys Pro Leu Lys Pro Pro Cys Asp Leu Ser Met Gln Ser
Val Glu Val
    50                  55                  60

Ala Gly Ser Gly Gly Ala Arg Arg Ser Ala Leu Leu Asp
Ser Asp Glu
65                  70                  75
        80

Pro Leu Val Tyr Phe Tyr Asp Asp Val Thr Thr Leu Tyr
Glu Gly Phe
                85                  90
    95

Gln Arg Gly Ile Gln Val Ser Asn Asn Gly Pro Cys Leu
Gly Ser Arg
            100                 105
110

Lys Pro Asp Gln Pro Tyr Glu Trp Leu Ser Tyr Lys Gln
Val Ala Glu
        115                 120                 125

Leu Ser Glu Cys Ile Gly Ser Ala Leu Ile Gln Lys Gly
Phe Lys Thr
    130                 135                 140

Ala Pro Asp Gln Phe Ile Gly Ile Phe Ala Gln Asn Arg
Pro Glu Trp
145                 150                 155
        160

Val Ile Ile Glu Gln Gly Cys Phe Ala Tyr Ser Met Val
Ile Val Pro
                    165                     170
    175

Leu Tyr Asp Thr Leu Gly Asn Glu Ala Ile Thr Tyr Ile
Val Asn Lys
                180                     185
190

Ala Glu Leu Ser Leu Val Phe Val Asp Lys Pro Glu Lys
Ala Lys Leu
            195                     200                     205

Leu Leu Glu Gly Val Glu Asn Lys Leu Ile Pro Gly Leu
Lys Ile Ile
    210                     215                     220

Val Val Met Asp Ala Tyr Gly Ser Glu Leu Val Glu Arg
Gly Gln Arg
225                     230                     235
            240

Cys Gly Val Glu Val Thr Ser Met Lys Ala Met Glu Asp
Leu Gly Arg
                    245                     250
    255

Ala Asn Arg Arg Lys Pro Lys Pro Pro Ala Pro Glu Asp
Leu Ala Val
                260                     265
270

Ile Cys Phe Thr Ser Gly Thr Thr Gly Asn Pro Lys Gly
Ala Met Val

275                     280                     285

Thr His Arg Asn Ile Val Ser Asp Cys Ser Ala Phe Val
Lys Ala Thr
    290                     295                     300

Glu Asn Thr Val Asn Pro Cys Pro Asp Asp Thr Leu Ile
Ser Phe Leu
305                     310                     315
        320

Pro Leu Ala His Met Phe Glu Arg Val Val Glu Cys Val
Met Leu Cys
                325                     330
    335

His Gly Ala Lys Ile Gly Phe Phe Gln Gly Asp Ile Arg
Leu Leu Met
            340                     345
350

Asp Asp Leu Lys Val Leu Gln Pro Thr Val Phe Pro Val
Val Pro Arg
        355                     360                     365

Leu Leu Asn Arg Met Phe Asp Arg Ile Phe Gly Gln Ala
Asn Thr Thr
    370                     375                     380

Leu Lys Arg Trp Leu Leu Asp Phe Ala Ser Lys Arg Lys
Glu Ala Glu
385                     390                     395
        400

Leu Arg Ser Gly Ile Ile Arg Asn Asn Ser Leu Trp Asp
Arg Leu Ile
                405                 410
    415

Phe His Lys Val Gln Ser Ser Leu Gly Gly Arg Val Arg
Leu Met Val
            420                 425
430

Thr Gly Ala Ala Pro Val Ser Ala Thr Val Leu Thr Phe
Leu Arg Ala
        435                 440                 445

Ala Leu Gly Cys Gln Phe Tyr Glu Gly Tyr Gly Gln Thr
Glu Cys Thr
    450                 455                 460

Ala Gly Cys Cys Leu Thr Met Pro Gly Asp Trp Thr Ala
Gly His Val
465                 470                 475
        480

Gly Ala Pro Met Pro Cys Asn Leu Ile Lys Leu Val Asp
Val Glu Glu
                485                 490
    495

Met Asn Tyr Met Ala Ala Glu Gly Glu Gly Glu Val Cys
Val Lys Gly
            500                 505
510

Pro Asn Val Phe Gln Gly Tyr Leu Lys Asp Pro Ala Lys
Thr Ala Glu
        515                 520                 525

Ala Leu Asp Lys Asp Gly Trp Leu His Thr Gly Asp Ile
Gly Lys Trp
    530                 535                 540

Leu Pro Asn Gly Thr Leu Lys Ile Ile Asp Arg Lys Lys
His Ile Phe
545                 550                 555
        560

Lys Leu Ala Gln Gly Glu Tyr Ile Ala Pro Glu Lys Ile
Glu Asn Ile
                565                 570
    575

Tyr Met Arg Ser Glu Pro Val Ala Gln Val Phe Val His
Gly Glu Ser
            580                 585
590

Leu Gln Ala Phe Leu Ile Ala Ile Val Val Pro Asp Val
Glu Thr Leu
        595                 600                 605

Cys Ser Trp Ala Gln Lys Arg Gly Phe Glu Gly Ser Phe
Glu Glu Leu
    610                 615                 620

Cys Arg Asn Lys Asp Val Lys Lys Ala Ile Leu Glu Asp
Met Val Arg
625                 630                 635
        640

Leu Gly Lys Asp Ser Gly Leu Lys Pro Phe Glu Gln Val
Lys Gly Ile

                    645                 650
    655

Thr Leu His Pro Glu Leu Phe Ser Ile Asp Asn Gly Leu
Leu Thr Pro
                660                 665
670

Thr Met Lys Ala Lys Arg Pro Glu Leu Arg Asn Tyr Phe
Arg Ser Gln
        675                 680                 685

Ile Asp Asp Leu Tyr Ser Thr Ile Lys Val
    690                 695

<210>  13
<211>  552
<212>  PRT
<213>  Homo sapiens

<400>  13

Met Leu Asp His Lys Asp Leu Glu Ala Glu Ile His Pro
Leu Lys Asn
1               5                   10
    15

Glu Glu Arg Lys Ser Gln Glu Asn Leu Gly Asn Pro Ser
Lys Asn Glu
            20                  25
30

Asp Asn Val Lys Ser Ala Pro Pro Gln Ser Arg Leu Ser
Arg Cys Arg
        35                  40                  45

Ala Ala Ala Phe Phe Leu Ser Leu Phe Leu Cys Leu Phe

Val Val Phe
    50                  55                  60

Val Val Ser Phe Val Ile Pro Cys Pro Asp Arg Pro Ala
Ser Gln Arg
65                  70                  75
        80

Met Trp Arg Ile Asp Tyr Ser Ala Ala Val Ile Tyr Asp
Phe Leu Ala
                85                  90
    95

Val Asp Asp Ile Asn Gly Asp Arg Ile Gln Asp Val Leu
Phe Leu Tyr
            100                 105
110

Lys Asn Thr Asn Ser Ser Asn Asn Phe Ser Arg Ser Cys
Val Asp Glu
        115                 120                 125

Gly Phe Ser Ser Pro Cys Thr Phe Ala Ala Ala Val Ser
Gly Ala Asn
    130                 135                 140

Gly Ser Thr Leu Trp Glu Arg Pro Val Ala Gln Asp Val
Ala Leu Val
145                 150                 155
        160

Glu Cys Ala Val Pro Gln Pro Arg Gly Ser Glu Ala Pro
Ser Ala Cys
                165                 170
    175

Ile Leu Val Gly Arg Pro Ser Ser Phe Ile Ala Val Asn
Leu Phe Thr
            180                 185
190

Gly Glu Thr Leu Trp Asn His Ser Ser Ser Phe Ser Gly
Asn Ala Ser
        195                 200                 205

Ile Leu Ser Pro Leu Leu Gln Val Pro Asp Val Asp Gly
Asp Gly Ala
    210                 215                 220

Pro Asp Leu Leu Val Leu Thr Gln Glu Arg Glu Glu Val
Ser Gly His
225                 230                 235
        240

Leu Tyr Ser Gly Ser Thr Gly His Gln Ile Gly Leu Arg
Gly Ser Leu
                245                 250
    255

Gly Val Asp Gly Glu Ser Gly Phe Leu Leu His Val Thr
Arg Thr Gly
            260                 265
270

Ala His Tyr Ile Leu Phe Pro Cys Ala Ser Ser Leu Cys
Gly Cys Ser
        275                 280                 285

Val Lys Gly Leu Tyr Glu Lys Val Thr Gly Ser Gly Gly
Pro Phe Lys
    290                 295                 300

Ser Asp Pro His Trp Glu Ser Met Leu Asn Ala Thr Thr
Arg Arg Met
305                 310                 315
        320

Leu Ser His Ser Ser Gly Ala Val Arg Tyr Leu Met His
Val Pro Gly
                325                 330
    335

Asn Ala Gly Ala Asp Val Leu Leu Val Gly Ser Glu Ala
Phe Val Leu
            340                 345
350

Leu Asp Gly Gln Glu Leu Thr Pro Arg Trp Thr Pro Lys
Ala Ala His
        355                 360                 365

Val Leu Arg Lys Pro Ile Phe Gly Arg Tyr Lys Pro Asp
Thr Leu Ala
    370                 375                 380

Val Ala Val Glu Asn Gly Thr Gly Thr Asp Arg Gln Ile
Leu Phe Leu
385                 390                 395
        400

Asp Leu Gly Thr Gly Ala Val Leu Cys Ser Leu Ala Leu
Pro Ser Leu
                405                 410
    415

Pro Gly Gly Pro Leu Ser Ala Ser Leu Pro Thr Ala Asp

His Arg Ser
                420                     425
430

Ala Phe Phe Phe Trp Gly Leu His Glu Leu Gly Ser Thr
Ser Glu Thr
            435                     440                     445

Glu Thr Gly Glu Ala Arg His Ser Leu Tyr Met Phe His
Pro Thr Leu
        450                     455                     460

Pro Arg Val Leu Leu Glu Leu Ala Asn Val Ser Thr His
Ile Val Ala
465                     470                     475
            480

Phe Asp Ala Val Leu Phe Glu Pro Ser Arg His Ala Ala
Tyr Ile Leu
                    485                     490
        495

Leu Thr Gly Pro Ala Asp Ser Glu Ala Pro Gly Leu Val
Ser Val Ile
                500                     505
510

Lys His Lys Val Arg Asp Leu Val Pro Ser Ser Arg Val
Val Arg Leu
            515                     520                     525

Gly Glu Gly Gly Pro Asp Ser Asp Gln Ala Ile Arg Asp
Arg Phe Ser
        530                     535                     540

Arg Leu Arg Tyr Gln Ser Glu Ala
545                 550

<210>  14
<211>  963
<212>  PRT
<213>  Homo sapiens

<400>  14

Met Gly Leu Pro Glu Pro Gly Pro Leu Arg Leu Leu Ala
Leu Leu Leu
1               5                   10
    15

Leu Leu Leu Leu Leu Leu Leu Leu Gln Leu Gln His Leu
Ala Ala Ala
            20                  25
30

Ala Ala Asp Pro Leu Leu Gly Gly Gln Gly Pro Ala Lys
Asp Cys Glu
        35                  40                  45

Lys Asp Gln Phe Gln Cys Arg Asn Glu Arg Cys Ile Pro
Ser Val Trp
    50                  55                  60

Arg Cys Asp Glu Asp Asp Asp Cys Leu Asp His Ser Asp
Glu Asp Asp
65                  70                  75
        80

Cys Pro Lys Lys Thr Cys Ala Asp Ser Asp Phe Thr Cys
Asp Asn Gly
                85                  90
    95

His Cys Ile His Glu Arg Trp Lys Cys Asp Gly Glu Glu
Glu Cys Pro
            100                 105
110

Asp Gly Ser Asp Glu Ser Glu Ala Thr Cys Thr Lys Gln
Val Cys Pro
        115                 120                 125

Ala Glu Lys Leu Ser Cys Gly Pro Thr Ser His Lys Cys
Val Pro Ala
    130                 135                 140

Ser Trp Arg Cys Asp Gly Glu Lys Asp Cys Glu Gly Gly
Ala Asp Glu
145                 150                 155
        160

Ala Gly Cys Ala Thr Leu Cys Ala Pro His Glu Phe Gln
Cys Gly Asn
                165                 170
    175

Arg Ser Cys Leu Ala Ala Val Phe Val Cys Asp Gly Asp
Asp Asp Cys
            180                 185
190

Gly Asp Gly Ser Asp Glu Arg Gly Cys Ala Asp Pro Ala
Cys Gly Pro
        195                 200                 205

Arg Glu Phe Arg Cys Gly Gly Asp Gly Gly Gly Ala Cys
Ile Pro Glu

210                 215                 220

Arg Trp Val Cys Asp Arg Gln Phe Asp Cys Glu Asp Arg
Ser Asp Glu
225                 230                 235
        240

Ala Ala Glu Leu Cys Gly Arg Pro Gly Pro Gly Ala Thr
Ser Ala Pro
                245                 250
    255

Ala Ala Cys Ala Thr Ala Ser Gln Phe Ala Cys Arg Ser
Gly Glu Cys
            260                 265
270

Val His Leu Gly Trp Arg Cys Asp Gly Asp Arg Asp Cys
Lys Asp Lys
        275                 280                 285

Ser Asp Glu Ala Asp Cys Pro Leu Gly Thr Cys Arg Gly
Asp Glu Phe
    290                 295                 300

Gln Cys Gly Asp Gly Thr Cys Val Leu Ala Ile Lys His
Cys Asn Gln
305                 310                 315
        320

Glu Gln Asp Cys Pro Asp Gly Ser Asp Glu Ala Gly Cys
Leu Gln Gly
                325                 330
    335

Leu Asn Glu Cys Leu His Asn Asn Gly Gly Cys Ser His
Ile Cys Thr
340 345
350

Asp Leu Lys Ile Gly Phe Glu Cys Thr Cys Pro Ala Gly
Phe Gln Leu
355 360 365

Leu Asp Gln Lys Thr Cys Gly Asp Ile Asp Glu Cys Lys
Asp Pro Asp
370 375 380

Ala Cys Ser Gln Ile Cys Val Asn Tyr Lys Gly Tyr Phe
Lys Cys Glu
385 390 395
400

Cys Tyr Pro Gly Tyr Glu Met Asp Leu Leu Thr Lys Asn
Cys Lys Ala
405 410
415

Ala Ala Gly Lys Ser Pro Ser Leu Ile Phe Thr Asn Arg
His Glu Val
420 425
430

Arg Arg Ile Asp Leu Val Lys Arg Asn Tyr Ser Arg Leu
Ile Pro Met
435 440 445

Leu Lys Asn Val Val Ala Leu Asp Val Glu Val Ala Thr
Asn Arg Ile
450 455 460

Tyr Trp Cys Asp Leu Ser Tyr Arg Lys Ile Tyr Ser Ala
Tyr Met Asp
465                 470                 475
      480

Lys Ala Ser Asp Pro Lys Glu Gln Glu Val Leu Ile Asp
Glu Gln Leu
            485                 490
    495

His Ser Pro Glu Gly Leu Ala Val Asp Trp Val His Lys
His Ile Tyr
          500                 505
510

Trp Thr Asp Ser Gly Asn Lys Thr Ile Ser Val Ala Thr
Val Asp Gly
      515                 520                 525

Gly Arg Arg Arg Thr Leu Phe Ser Arg Asn Leu Ser Glu
Pro Arg Ala
    530                 535                 540

Ile Ala Val Asp Pro Leu Arg Gly Phe Met Tyr Trp Ser
Asp Trp Gly
545                 550                 555
      560

Asp Gln Ala Lys Ile Glu Lys Ser Gly Leu Asn Gly Val
Asp Arg Gln
            565                 570
    575

Thr Leu Val Ser Asp Asn Ile Glu Trp Pro Asn Gly Ile
Thr Leu Asp

                580                     585
590

Leu Leu Ser Gln Arg Leu Tyr Trp Val Asp Ser Lys Leu
His Gln Leu
        595                     600                     605

Ser Ser Ile Asp Phe Ser Gly Gly Asn Arg Lys Thr Leu
Ile Ser Ser
    610                     615                     620

Thr Asp Phe Leu Ser His Pro Phe Gly Ile Ala Val Phe
Glu Asp Lys
625                     630                     635
        640

Val Phe Trp Thr Asp Leu Glu Asn Glu Ala Ile Phe Ser
Ala Asn Arg
                    645                     650
    655

Leu Asn Gly Leu Glu Ile Ser Ile Leu Ala Glu Asn Leu
Asn Asn Pro
                660                     665
670

His Asp Ile Val Ile Phe His Glu Leu Lys Gln Pro Arg
Ala Pro Asp
        675                     680                     685

Ala Cys Glu Leu Ser Val Gln Pro Asn Gly Gly Cys Glu
Tyr Leu Cys
    690                     695                     700

Leu Pro Ala Pro Gln Ile Ser Ser His Ser Pro Lys Tyr
Thr Cys Ala
705                 710                 715
        720

Cys Pro Asp Thr Met Trp Leu Gly Pro Asp Met Lys Arg
Cys Tyr Arg
                725                 730
    735

Ala Pro Gln Ser Thr Ser Thr Thr Thr Leu Ala Ser Thr
Met Thr Arg
            740                 745
750

Thr Val Pro Ala Thr Thr Arg Ala Pro Gly Thr Thr Val
His Arg Ser
        755                 760                 765

Thr Tyr Gln Asn His Ser Thr Glu Thr Pro Ser Leu Thr
Ala Ala Val
    770                 775                 780

Pro Ser Ser Val Ser Val Pro Arg Ala Pro Ser Ile Ser
Pro Ser Thr
785                 790                 795
        800

Leu Ser Pro Ala Thr Ser Asn His Ser Gln His Tyr Ala
Asn Glu Asp
                805                 810
    815

Ser Lys Met Gly Ser Thr Val Thr Ala Ala Val Ile Gly
Ile Ile Val
            820                 825
830

Pro Ile Val Val Ile Ala Leu Leu Cys Met Ser Gly Tyr
Leu Ile Trp
        835                 840                 845

Arg Asn Trp Lys Arg Lys Asn Thr Lys Ser Met Asn Phe
Asp Asn Pro
    850                 855                 860

Val Tyr Arg Lys Thr Thr Glu Glu Glu Asp Glu Asp Glu
Leu His Ile
865                 870                 875
        880

Gly Arg Thr Ala Gln Ile Gly His Val Tyr Pro Ala Ala
Ile Ser Ser
                885                 890
    895

Phe Asp Arg Pro Leu Trp Ala Glu Pro Cys Leu Gly Glu
Thr Arg Glu
            900                 905
910

Pro Glu Asp Pro Ala Pro Ala Leu Lys Glu Leu Phe Val
Leu Pro Gly
        915                 920                 925

Glu Pro Arg Ser Gln Leu His Gln Leu Pro Lys Asn Pro
Leu Ser Glu
    930                 935                 940

Leu Pro Val Val Lys Ser Lys Arg Val Ala Leu Ser Leu
Glu Asp Asp

945                 950                 955
        960

Gly Leu Pro

<210>  15
<211>  150
<212>  PRT
<213>  Homo sapiens

<400>  15

Met Ser Gly Ser Met Ala Thr Ala Glu Ala Ser Gly Ser
Asp Gly Lys
1               5                   10
    15

Gly Gln Glu Val Glu Thr Ser Val Thr Tyr Tyr Arg Leu
Glu Glu Val
            20                  25
30

Ala Lys Arg Asn Ser Leu Lys Glu Leu Trp Leu Val Ile
His Gly Arg
        35                  40                  45

Val Tyr Asp Val Thr Arg Phe Leu Asn Glu His Pro Gly
Gly Glu Glu
    50                  55                  60

Val Leu Leu Glu Gln Ala Gly Val Asp Ala Ser Glu Ser
Phe Glu Asp
65                  70                  75
        80

Val Gly His Ser Ser Asp Ala Arg Glu Met Leu Lys Gln

Tyr Tyr Ile
                85                  90
    95

Gly Asp Ile His Pro Ser Asp Leu Lys Pro Glu Ser Gly
Ser Lys Asp
            100                 105
110

Pro Ser Lys Asn Asp Thr Cys Lys Ser Cys Trp Ala Tyr
Trp Ile Leu
        115                 120                 125

Pro Ile Ile Gly Ala Val Leu Leu Gly Phe Leu Tyr Arg
Tyr Tyr Thr
    130                 135                 140

Ser Glu Ser Lys Ser Ser
145                 150

<210>  16
<211>  327
<212>  PRT
<213>  Homo sapiens

<400>  16

Met Ala Ala Ala Ala Ala Ala Ala Ala Ala Thr Asn Gly
Thr Gly Gly
1               5                   10
    15

Ser Ser Gly Met Glu Val Asp Ala Ala Val Val Pro Ser
Val Met Ala
            20                  25
30

Cys Gly Val Thr Gly Ser Val Ser Val Ala Leu His Pro
Leu Val Ile
        35                  40                  45

Leu Asn Ile Ser Asp His Trp Ile Arg Met Arg Ser Gln
Glu Gly Arg
    50                  55                  60

Pro Val Gln Val Ile Gly Ala Leu Ile Gly Lys Gln Glu
Gly Arg Asn
65                  70                  75
        80

Ile Glu Val Met Asn Ser Phe Glu Leu Leu Ser His Thr
Val Glu Glu
                85                  90
    95

Lys Ile Ile Ile Asp Lys Glu Tyr Tyr Tyr Thr Lys Glu
Glu Gln Phe
            100                 105
110

Lys Gln Val Phe Lys Glu Leu Glu Phe Leu Gly Trp Tyr
Thr Thr Gly
        115                 120                 125

Gly Pro Pro Asp Pro Ser Asp Ile His Val His Lys Gln
Val Cys Glu
    130                 135                 140

Ile Ile Glu Ser Pro Leu Phe Leu Lys Leu Asn Pro Met
Thr Lys His
145                 150                 155
        160

Thr Asp Leu Pro Val Ser Val Phe Glu Ser Val Ile Asp
Ile Ile Asn
                165                     170
    175

Gly Glu Ala Thr Met Leu Phe Ala Glu Leu Thr Tyr Thr
Leu Ala Thr
            180                     185
190

Glu Glu Ala Glu Arg Ile Gly Val Asp His Val Ala Arg
Met Thr Ala
        195                     200                     205

Thr Gly Ser Gly Glu Asn Ser Thr Val Ala Glu His Leu
Ile Ala Gln
    210                     215                     220

His Ser Ala Ile Lys Met Leu His Ser Arg Val Lys Leu
Ile Leu Glu
225                     230                     235
        240

Tyr Val Lys Ala Ser Glu Ala Gly Glu Val Pro Phe Asn
His Glu Ile
                245                     250
    255

Leu Arg Glu Ala Tyr Ala Leu Cys His Cys Leu Pro Val
Leu Ser Thr
            260                     265
270

Asp Lys Phe Lys Thr Asp Phe Tyr Asp Gln Cys Asn Asp
Val Gly Leu

275                 280                 285

Met Ala Tyr Leu Gly Thr Ile Thr Lys Thr Cys Asn Thr
Met Asn Gln
    290                 295                 300

Phe Val Asn Lys Phe Asn Val Leu Tyr Asp Arg Gln Gly
Ile Gly Arg
305                 310                 315
        320

Arg Met Arg Gly Leu Phe Phe
                325

<210>  17
<211>  343
<212>  PRT
<213>  Homo sapiens

<400>  17

Met Ala Gln Lys Gly Val Leu Gly Pro Gly Gln Leu Gly
Ala Val Ala
1               5                   10
    15

Ile Leu Leu Tyr Leu Gly Leu Leu Arg Ser Gly Thr Gly
Lys Glu Gly
            20                  25
30

Ala Ala Ala Pro Cys Gly Val Ala Pro Gln Ala Arg Ile
Thr Gly Gly
        35                  40                  45

Ser Ser Ala Val Ala Gly Gln Trp Pro Trp Gln Val Ser

Ile Thr Tyr
    50                  55                  60

Glu Gly Val His Val Cys Gly Gly Ser Leu Val Ser Glu
Gln Trp Val
65                  70                  75
        80

Leu Ser Ala Ala His Cys Phe Pro Ser Glu His His Lys
Glu Ala Tyr
                85                  90
    95

Glu Val Lys Leu Gly Ala His Gln Leu Asp Ser Tyr Ser
Glu Asp Ala
            100                 105
110

Lys Val Ser Thr Leu Lys Asp Ile Ile Pro His Pro Ser
Tyr Leu Gln
        115                 120                 125

Glu Gly Ser Gln Gly Asp Ile Ala Leu Leu Gln Leu Ser
Arg Pro Ile
    130                 135                 140

Thr Phe Ser Arg Tyr Ile Arg Pro Ile Cys Leu Pro Ala
Ala Asn Ala
145                 150                 155
        160

Ser Phe Pro Asn Gly Leu His Cys Thr Val Thr Gly Trp
Gly His Val
                165                 170
    175

Ala Pro Ser Val Ser Leu Leu Thr Pro Lys Pro Leu Gln
Gln Leu Glu
            180                     185
190

Val Pro Leu Ile Ser Arg Glu Thr Cys Asn Cys Leu Tyr
Asn Ile Asp
        195                     200                     205

Ala Lys Pro Glu Glu Pro His Phe Val Gln Glu Asp Met
Val Cys Ala
    210                     215                     220

Gly Tyr Val Glu Gly Gly Lys Asp Ala Cys Gln Gly Asp
Ser Gly Gly
225                     230                     235
        240

Pro Leu Ser Cys Pro Val Glu Gly Leu Trp Tyr Leu Thr
Gly Ile Val
                245                     250
    255

Ser Trp Gly Asp Ala Cys Gly Ala Arg Asn Arg Pro Gly
Val Tyr Thr
            260                     265
270

Leu Ala Ser Ser Tyr Ala Ser Trp Ile Gln Ser Lys Val
Thr Glu Leu
        275                     280                     285

Gln Pro Arg Val Val Pro Gln Thr Gln Glu Ser Gln Pro
Asp Ser Asn
    290                     295                     300

Leu Cys Gly Ser His Leu Ala Phe Ser Ser Ala Pro Ala
Gln Gly Leu
305                 310                 315
        320

Leu Arg Pro Ile Leu Phe Leu Pro Leu Gly Leu Ala Leu
Gly Leu Leu
                325                 330
    335

Ser Pro Trp Leu Ser Glu His
            340

<210>  18
<211>  198
<212>  PRT
<213>  Homo sapiens

<400>  18

Met Ala Pro Ala Arg Leu Phe Ala Leu Leu Leu Phe Phe
Val Gly Gly
1               5                   10
    15

Val Ala Glu Ser Ile Arg Glu Thr Glu Val Ile Asp Pro
Gln Asp Leu
            20                  25
30

Leu Glu Gly Arg Tyr Phe Ser Gly Ala Leu Pro Asp Asp
Glu Asp Val
        35                  40                  45

Val Gly Pro Gly Gln Glu Ser Asp Asp Phe Glu Leu Ser
Gly Ser Gly

50                  55                  60

Asp Leu Asp Asp Leu Glu Asp Ser Met Ile Gly Pro Glu
Val Val His
65                  70                  75
    80

Pro Leu Val Pro Leu Asp Asn His Ile Pro Glu Arg Ala
Gly Ser Gly
                85                  90
    95

Ser Gln Val Pro Thr Glu Pro Lys Lys Leu Glu Glu Asn
Glu Val Ile
            100                 105
110

Pro Lys Arg Ile Ser Pro Val Glu Glu Ser Glu Asp Val
Ser Asn Lys
        115                 120                 125

Val Ser Met Ser Ser Thr Val Gln Gly Ser Asn Ile Phe
Glu Arg Thr
    130                 135                 140

Glu Val Leu Ala Ala Leu Ile Val Gly Gly Ile Val Gly
Ile Leu Phe
145                 150                 155
        160

Ala Val Phe Leu Ile Leu Leu Leu Met Tyr Arg Met Lys
Lys Lys Asp
                165                 170
    175

Glu Gly Ser Tyr Asp Leu Gly Lys Lys Pro Ile Tyr Lys
Lys Ala Pro
            180                 185
190

Thr Asn Glu Phe Tyr Ala
        195

<210>  19
<211>  492
<212>  PRT
<213>  Homo sapiens

<400>  19

<210>  20
<211>  180
<212>  PRT
<213>  Homo sapiens

<400>  20

Met Ala Ser Thr Ser Tyr Asp Tyr Cys Arg Val Pro Met
Glu Asp Gly
1               5                   10
    15

Asp Lys Arg Cys Lys Leu Leu Leu Gly Ile Gly Ile Leu
Val Leu Leu
            20                  25
30

Ile Ile Val Ile Leu Gly Val Pro Leu Ile Ile Phe Thr
Ile Lys Ala
        35                  40                  45

Asn Ser Glu Ala Cys Arg Asp Gly Leu Arg Ala Val Met
Glu Cys Arg
    50                  55                  60

Asn Val Thr His Leu Leu Gln Gln Glu Leu Thr Glu Ala
Gln Lys Gly
65                  70                  75
        80

Phe Gln Asp Val Glu Ala Gln Ala Ala Thr Cys Asn His
Thr Val Met
                85                  90

    95

Ala Leu Met Ala Ser Leu Asp Ala Glu Lys Ala Gln Gly
Gln Lys Lys
            100                 105

110

Val Glu Glu Leu Glu Gly Glu Ile Thr Thr Leu Asn His
Lys Leu Gln
        115                 120                 125

Asp Ala Ser Ala Glu Val Glu Arg Leu Arg Arg Glu Asn
Gln Val Leu
    130                 135                 140

Ser Val Arg Ile Ala Asp Lys Lys Tyr Tyr Pro Ser Ser
Gln Asp Ser
145                 150                 155
        160

Ser Ser Ala Ala Ala Pro Gln Leu Leu Ile Val Leu Leu
Gly Leu Ser
                165                 170

    175

Ala Leu Leu Gln
            180

<210>  21
<211>  341
<212>  PRT
<213>  Homo sapiens

<400>  21

Met Arg Lys Val Val Leu Ile Thr Gly Ala Ser Ser Gly
Ile Gly Leu
1               5                   10
    15

Ala Leu Cys Lys Arg Leu Leu Ala Glu Asp Asp Glu Leu
His Leu Cys
            20                  25
30

Leu Ala Cys Arg Asn Met Ser Lys Ala Glu Ala Val Cys
Ala Ala Leu
        35                  40                  45

Leu Ala Ser His Pro Thr Ala Glu Val Thr Ile Val Gln
Val Asp Val
    50                  55                  60

Ser Asn Leu Gln Ser Val Phe Arg Ala Ser Lys Glu Leu
Lys Gln Arg
65                  70                  75
        80

Phe Gln Arg Leu Asp Cys Ile Tyr Leu Asn Ala Gly Ile
Met Pro Asn
                85                  90
    95

Pro Gln Leu Asn Ile Lys Ala Leu Phe Phe Gly Leu Phe

Ser Arg Lys
                100                 105
110

Val Ile His Met Phe Ser Thr Ala Glu Gly Leu Leu Thr
Gln Gly Asp
            115                 120                 125

Lys Ile Thr Ala Asp Gly Leu Gln Glu Val Phe Glu Thr
Asn Val Phe
    130                 135                 140

Gly His Phe Ile Leu Ile Arg Glu Leu Glu Pro Leu Leu
Cys His Ser
145                 150                 155
            160

Asp Asn Pro Ser Gln Leu Ile Trp Thr Ser Ser Arg Ser
Ala Arg Lys
                165                 170
    175

Ser Asn Phe Ser Leu Glu Asp Phe Gln His Ser Lys Gly
Lys Glu Pro
                180                 185
190

Tyr Ser Ser Ser Lys Tyr Ala Thr Asp Leu Leu Ser Val
Ala Leu Asn
            195                 200                 205

Arg Asn Phe Asn Gln Gln Gly Leu Tyr Ser Asn Val Ala
Cys Pro Gly
    210                 215                 220

Thr Ala Leu Thr Asn Leu Thr Tyr Gly Ile Leu Pro Pro
Phe Ile Trp
225                 230                 235
        240

Thr Leu Leu Met Pro Ala Ile Leu Leu Leu Arg Phe Phe
Ala Asn Ala
                245                 250
    255

Phe Thr Leu Thr Pro Tyr Asn Gly Thr Glu Ala Leu Val
Trp Leu Phe
            260                 265
270

His Gln Lys Pro Glu Ser Leu Asn Pro Leu Ile Lys Tyr
Leu Ser Ala
        275                 280                 285

Thr Thr Gly Phe Gly Arg Asn Tyr Ile Met Thr Gln Lys
Met Asp Leu
    290                 295                 300

Asp Glu Asp Thr Ala Glu Lys Phe Tyr Gln Lys Leu Leu
Glu Leu Glu
305                 310                 315
        320

Lys His Ile Arg Val Thr Ile Gln Lys Thr Asp Asn Gln
Ala Arg Leu
                325                 330
    335

Ser Gly Ser Cys Leu
            340

<210>  22
<211>  454
<212>  PRT
<213>  Homo sapiens

<400>  22

<210>  23
<211>  528
<212>  PRT
<213>  Homo sapiens

<400>  23

Met Arg Cys Ala Leu Ala Leu Ser Ala Leu Leu Leu Leu
Leu Ser Thr
1               5                   10
    15

Pro Pro Leu Leu Pro Ser Ser Pro Ser Pro Ser Pro Ser
Pro Ser Gln
            20                  25
30

Asn Glu Thr Ala Thr Gln Thr Thr Thr Asp Ser Ser Asn
Lys Thr Ala
        35                  40                  45

Pro Thr Pro Ala Ser Ser Val Thr Ile Met Ala Thr Asp
Thr Ala Gln
    50                  55                  60

Gln Ser Thr Val Pro Thr Ser Lys Ala Asn Glu Ile Leu
Ala Ser Val
65                  70                  75
        80

Lys Ala Thr Thr Leu Gly Val Ser Ser Asp Ser Pro Gly
Thr Thr Thr
                85                  90
    95

Leu Ala Gln Gln Val Ser Gly Pro Val Asn Thr Thr Val
Ala Arg Gly
            100                 105
110

Gly Gly Ser Gly Asn Pro Thr Thr Thr Ile Glu Ser Pro

Lys Ser Thr
        115                 120                 125

Lys Ser Ala Asp Thr Thr Thr Val Ala Thr Ser Thr Ala
Thr Ala Lys
    130                 135                 140

Pro Asn Thr Thr Ser Ser Gln Asn Gly Ala Glu Asp Thr
Thr Asn Ser
145                 150                 155
        160

Gly Gly Lys Ser Ser His Ser Val Thr Thr Asp Leu Thr
Ser Thr Lys
                165                 170
    175

Ala Glu His Leu Thr Thr Pro His Pro Thr Ser Pro Leu
Ser Pro Arg
            180                 185
190

Gln Pro Thr Ser Thr His Pro Val Ala Thr Pro Thr Ser
Ser Gly His
        195                 200                 205

Asp His Leu Met Lys Ile Ser Ser Ser Ser Ser Thr Val
Ala Ile Pro
    210                 215                 220

Gly Tyr Thr Phe Thr Ser Pro Gly Met Thr Thr Thr Leu
Pro Ser Ser
225                 230                 235
        240

Val Ile Ser Gln Arg Thr Gln Gln Thr Ser Ser Gln Met
Pro Ala Ser
245 250
255

Ser Thr Ala Pro Ser Ser Gln Glu Thr Val Gln Pro Thr
Ser Pro Ala
260 265
270

Thr Ala Leu Arg Thr Pro Thr Leu Pro Glu Thr Met Ser
Ser Ser Pro
275 280 285

Thr Ala Ala Ser Thr Thr His Arg Tyr Pro Lys Thr Pro
Ser Pro Thr
290 295 300

Val Ala His Glu Ser Asn Trp Val Thr Pro Ala Gly Val
Gly Thr Gln
305 310 315
320

Thr Arg Val Glu Glu Ala Leu Arg Gln Ala Leu Thr His
Ser Leu Leu
325 330
335

Pro Ala Gly Gly Ala Ser Asp Glu Lys Leu Ile Ser Leu
Ile Cys Arg
340 345
350

Ala Val Lys Ala Thr Phe Asn Pro Ala Gln Asp Lys Cys
Gly Ile Arg
355 360 365

Leu Ala Ser Val Pro Gly Ser Gln Thr Val Val Val Lys
Glu Ile Thr
    370                 375                 380

Ile His Thr Lys Leu Pro Ala Lys Asp Val Tyr Glu Arg
Leu Lys Asp
385                 390                 395
        400

Lys Trp Asp Glu Leu Lys Glu Ala Gly Val Ser Asp Met
Lys Leu Gly
                405                 410
    415

Asp Gln Gly Pro Pro Glu Glu Ala Glu Asp Arg Phe Ser
Met Pro Leu
            420                 425
430

Ile Ile Thr Ile Val Cys Met Ala Ser Phe Leu Leu Leu
Val Ala Ala
        435                 440                 445

Leu Tyr Gly Cys Cys His Gln Arg Leu Ser Gln Arg Lys
Asp Gln Gln
    450                 455                 460

Arg Leu Thr Glu Glu Leu Gln Thr Val Glu Asn Gly Tyr
His Asp Asn
465                 470                 475
        480

Pro Thr Leu Glu Val Met Glu Thr Ser Ser Glu Met Gln

Glu Lys Lys
                485                 490
    495

Val Val Ser Leu Asn Gly Glu Leu Gly Asp Ser Trp Ile
Val Pro Leu
            500                 505
510

Asp Asn Leu Thr Lys Asp Asp Leu Asp Glu Glu Glu Asp
Thr His Leu
        515                 520                 525

<210>  24
<211>  475
<212>  PRT
<213>  Homo sapiens

<400>  24

<210>  25
<211>  1241
<212>  PRT
<213>  Homo sapiens

<400>  25

Met Ser Ser Ala Pro Arg Arg Pro Ala Lys Gly Ala Asp
Ser Phe Cys
1               5                   10
    15

Thr Pro Glu Pro Glu Ser Leu Gly Pro Gly Thr Pro Gly
Phe Pro Glu
            20                  25
30

Gln Glu Glu Asp Glu Leu His Arg Thr Leu Gly Val Glu
Arg Phe Glu
        35                  40                  45

Glu Ile Leu Gln Glu Ala Gly Ser Arg Gly Gly Glu Glu
Pro Gly Arg
    50                  55                  60

Ser Tyr Gly Glu Glu Asp Phe Glu Tyr His Arg Gln Ser
Ser His His
65                  70                  75
        80

Ile His His Pro Leu Ser Thr His Leu Pro Pro Asp Ala
Arg Arg Arg
                85                  90
    95

Lys Thr Pro Gln Gly Pro Gly Arg Lys Pro Arg Arg Arg
Pro Gly Ala
            100                 105
110

Ser Pro Thr Gly Glu Thr Pro Thr Ile Glu Glu Gly Glu
Glu Asp Glu
        115                 120                 125

Asp Glu Ala Ser Glu Ala Glu Gly Ala Arg Ala Leu Thr
Gln Pro Ser
    130                 135                 140

Pro Val Ser Thr Pro Ser Ser Val Gln Phe Phe Leu Gln
Glu Asp Asp
145                 150                 155
        160

Ser Ala Asp Arg Lys Ala Glu Arg Thr Ser Pro Ser Ser
Pro Ala Pro
                165                 170
    175

Leu Pro His Gln Glu Ala Thr Pro Arg Ala Ser Lys Gly

Ala Gln Ala
180 185
190

Gly Thr Gln Val Glu Glu Ala Glu Ala Glu Ala Val Ala
Val Ala Ser
195 200 205

Gly Thr Ala Gly Gly Asp Asp Gly Gly Ala Ser Gly Arg
Pro Leu Pro
210 215 220

Lys Ala Gln Pro Gly His Arg Ser Tyr Asn Leu Gln Glu
Arg Arg Arg
225 230 235
240

Ile Gly Ser Met Thr Gly Ala Glu Gln Ala Leu Leu Pro
Arg Val Pro
245 250
255

Thr Asp Glu Ile Glu Ala Gln Thr Leu Ala Thr Ala Asp
Leu Asp Leu
260 265
270

Met Lys Ser His Arg Phe Glu Asp Val Pro Gly Val Arg
Arg His Leu
275 280 285

Val Arg Lys Asn Ala Lys Gly Ser Thr Gln Ser Gly Arg
Glu Gly Arg
290 295 300

Glu Pro Gly Pro Thr Pro Arg Ala Arg Pro Arg Ala Pro
His Lys Pro
305                 310                 315
        320

His Glu Val Phe Val Glu Leu Asn Glu Leu Leu Leu Asp
Lys Asn Gln
                325                 330
    335

Glu Pro Gln Trp Arg Glu Thr Ala Arg Trp Ile Lys Phe
Glu Glu Asp
            340                 345
350

Val Glu Glu Glu Thr Glu Arg Trp Gly Lys Pro His Val
Ala Ser Leu
        355                 360                 365

Ser Phe Arg Ser Leu Leu Glu Leu Arg Arg Thr Leu Ala
His Gly Ala
    370                 375                 380

Val Leu Leu Asp Leu Asp Gln Gln Thr Leu Pro Gly Val
Ala His Gln
385                 390                 395
        400

Val Val Glu Gln Met Val Ile Ser Asp Gln Ile Lys Ala
Glu Asp Arg
                405                 410
    415

Ala Asn Val Leu Arg Ala Leu Leu Leu Lys His Ser His
Pro Ser Asp
            420                 425

430

Glu Lys Asp Phe Ser Phe Pro Arg Asn Ile Ser Ala Gly
Ser Leu Gly
        435                 440                 445

Ser Leu Leu Gly His His His Gly Gln Gly Ala Glu Ser
Asp Pro His
    450                 455                 460

Val Thr Glu Pro Leu Met Gly Gly Val Pro Glu Thr Arg
Leu Glu Val
465                 470                 475
        480

Glu Arg Glu Arg Glu Leu Pro Pro Pro Ala Pro Pro Ala
Gly Ile Thr
                485                 490
    495

Arg Ser Lys Ser Lys His Glu Leu Lys Leu Leu Glu Lys
Ile Pro Glu
            500                 505
510

Asn Ala Glu Ala Thr Val Val Leu Val Gly Cys Val Glu
Phe Leu Ser
        515                 520                 525

Arg Pro Thr Met Ala Phe Val Arg Leu Arg Glu Ala Val
Glu Leu Asp
    530                 535                 540

Ala Val Leu Glu Val Pro Val Pro Val Arg Phe Leu Phe

Leu Leu Leu
545                     550                     555
        560

Gly Pro Ser Ser Ala Asn Met Asp Tyr His Glu Ile Gly
Arg Ser Ile
                565                     570
    575

Ser Thr Leu Met Ser Asp Lys Gln Phe His Glu Ala Ala
Tyr Leu Ala
            580                     585
590

Asp Glu Arg Glu Asp Leu Leu Thr Ala Ile Asn Ala Phe
Leu Asp Cys
        595                     600                     605

Ser Val Val Leu Pro Pro Ser Glu Val Gln Gly Glu Glu
Leu Leu Arg
    610                     615                     620

Ser Val Ala His Phe Gln Arg Gln Met Leu Lys Lys Arg
Glu Glu Gln
625                     630                     635
        640

Gly Arg Leu Leu Pro Thr Gly Ala Gly Leu Glu Pro Lys
Ser Ala Gln
                645                     650
    655

Asp Lys Ala Leu Leu Gln Met Val Glu Ala Ala Gly Ala
Ala Glu Asp
            660                     665
670

Asp Pro Leu Arg Arg Thr Gly Arg Pro Phe Gly Gly Leu
Ile Arg Asp
        675                 680                 685

Val Arg Arg Arg Tyr Pro His Tyr Leu Ser Asp Phe Arg
Asp Ala Leu
    690                 695                 700

Asp Pro Gln Cys Leu Ala Ala Val Ile Phe Ile Tyr Phe
Ala Ala Leu
705                 710                 715
        720

Ser Pro Ala Ile Thr Phe Gly Gly Leu Leu Gly Glu Lys
Thr Gln Asp
                725                 730
    735

Leu Ile Gly Val Ser Glu Leu Ile Met Ser Thr Ala Leu
Gln Gly Val
            740                 745
750

Val Phe Cys Leu Leu Gly Ala Gln Pro Leu Leu Val Ile
Gly Phe Ser
        755                 760                 765

Gly Pro Leu Leu Val Phe Glu Glu Ala Phe Phe Ser Phe
Cys Ser Ser
    770                 775                 780

Asn His Leu Glu Tyr Leu Val Gly Arg Val Trp Ile Gly
Phe Trp Leu
785                 790                 795

800

Val Phe Leu Ala Leu Leu Met Val Ala Leu Glu Gly Ser
Phe Leu Val
                805                     810
    815

Arg Phe Val Ser Arg Phe Thr Gln Glu Ile Phe Ala Phe
Leu Ile Ser
            820                     825
830

Leu Ile Phe Ile Tyr Glu Thr Phe Tyr Lys Leu Val Lys
Ile Phe Gln
        835                     840                     845

Glu His Pro Leu His Gly Cys Ser Ala Ser Asn Ser Ser
Glu Val Asp
    850                     855                     860

Gly Gly Glu Asn Met Thr Trp Ala Gly Ala Arg Pro Thr
Leu Gly Pro
865                     870                     875
        880

Gly Asn Arg Ser Leu Ala Gly Gln Ser Gly Gln Gly Lys
Pro Arg Gly
                885                     890
    895

Gln Pro Asn Thr Ala Leu Leu Ser Leu Val Leu Met Ala
Gly Thr Phe
            900                     905
910

Phe Ile Ala Phe Phe Leu Arg Lys Phe Lys Asn Ser Arg

Phe Phe Pro
        915                 920                 925

Gly Arg Ile Arg Arg Val Ile Gly Asp Phe Gly Val Pro
Ile Ala Ile
    930                 935                 940

Leu Ile Met Val Leu Val Asp Tyr Ser Ile Glu Asp Thr
Tyr Thr Gln
945                 950                 955
        960

Lys Leu Ser Val Pro Ser Gly Phe Ser Val Thr Ala Pro
Glu Lys Arg
                965                 970
    975

Gly Trp Val Ile Asn Pro Leu Gly Glu Lys Ser Pro Phe
Pro Val Trp
            980                 985
990

Met Met Val Ala Ser Leu Leu Pro  Ala Ile Leu Val
Phe  Ile Leu Ile
        995                 1000
1005

Phe Met  Glu Thr Gln Ile Thr  Thr Leu Ile Ile Ser
Lys Lys Glu
    1010                1015                1020

Arg Met  Leu Gln Lys Gly Ser  Gly Phe His Leu Asp
Leu Leu Leu
    1025                1030                1035

Ile Val  Ala Met Gly Gly Ile  Cys Ala Leu Phe Gly
Leu Pro Trp
    1040                 1045                 1050

Leu Ala  Ala Ala Thr Val Arg  Ser Val Thr His Ala
Asn Ala Leu
    1055                 1060                 1065

Thr Val  Met Ser Lys Ala Val  Ala Pro Gly Asp Lys
Pro Lys Ile
    1070                 1075                 1080

Gln Glu  Val Lys Glu Gln Arg  Val Thr Gly Leu Leu
Val Ala Leu
    1085                 1090                 1095

Leu Val  Gly Leu Ser Ile Val  Ile Gly Asp Leu Leu
Arg Gln Ile
    1100                 1105                 1110

Pro Leu  Ala Val Leu Phe Gly  Ile Phe Leu Tyr Met
Gly Val Thr
    1115                 1120                 1125

Ser Leu  Asn Gly Ile Gln Phe  Tyr Glu Arg Leu His
Leu Leu Leu
    1130                 1135                 1140

Met Pro  Pro Lys His His Pro  Asp Val Thr Tyr Val
Lys Lys Val
    1145                 1150                 1155

Arg Thr  Leu Arg Met His Leu  Phe Thr Ala Leu Gln
Leu Leu Cys
    1160                1165                1170

Leu Ala  Leu Leu Trp Ala Val  Met Ser Thr Ala Ala
Ser Leu Ala
    1175                1180                1185

Phe Pro  Phe Ile Leu Ile Leu  Thr Val Pro Leu Arg
Met Val Val
    1190                1195                1200

Leu Thr  Arg Ile Phe Thr Asp  Arg Glu Met Lys Cys
Leu Asp Ala
    1205                1210                1215

Asn Glu  Ala Glu Pro Val Phe  Asp Glu Arg Glu Gly
Val Asp Glu
    1220                1225                1230

Tyr Asn  Glu Met Pro Met Pro  Val
    1235                1240

<210>  26
<211>  707
<212>  PRT
<213>  Homo sapiens

<400>  26

Met Ala Ala Ala Val Ala Ala Ala Gly Arg Leu Gly Trp
Leu Phe Ala

1               5                   10
    15

Ala Leu Cys Leu Gly Asn Ala Ala Gly Glu Ala Ala Pro
Gly Pro Arg
            20                  25
30

Val Leu Gly Phe Cys Leu Glu Glu Asp Gly Ala Ala Gly
Ala Gly Trp
        35                  40                  45

Val Arg Gly Gly Ala Ala Arg Asp Thr Pro Asp Ala Thr
Phe Leu Leu
    50                  55                  60

Arg Leu Phe Gly Pro Gly Phe Ala Asn Ser Ser Trp Ser
Trp Val Ala
65                  70                  75
        80

Pro Glu Gly Ala Gly Cys Arg Glu Glu Ala Ala Ser Pro
Ala Gly Glu
                85                  90
    95

Trp Arg Ala Leu Leu Arg Leu Arg Leu Arg Ala Glu Ala
Val Arg Pro
            100                 105
110

His Ser Ala Leu Leu Ala Val Arg Val Glu Pro Gly Gly
Gly Ala Ala
        115                 120                 125

Glu Glu Ala Ala Pro Pro Trp Ala Leu Gly Leu Gly Ala
Ala Gly Leu
    130                 135                 140

Leu Ala Leu Ala Ala Leu Ala Arg Gly Leu Gln Leu Ser
Ala Leu Ala
145                 150                 155
        160

Leu Ala Pro Ala Glu Val Gln Val Leu Arg Glu Ser Gly
Ser Glu Ala
                165                 170
    175

Glu Arg Ala Ala Ala Arg Arg Leu Glu Pro Ala Arg Arg
Trp Ala Gly
            180                 185
190

Cys Ala Leu Gly Ala Leu Leu Leu Leu Ala Ser Leu Ala
Gln Ala Ala
        195                 200                 205

Leu Ala Val Leu Leu Tyr Arg Ala Ala Gly Gln Arg Ala
Val Pro Ala
    210                 215                 220

Val Leu Gly Ser Ala Gly Leu Val Phe Leu Val Gly Glu
Val Val Pro
225                 230                 235
        240

Ala Ala Val Ser Gly Arg Trp Thr Leu Ala Leu Ala Pro
Arg Ala Leu
                245                 250
    255

Gly Leu Ser Arg Leu Ala Val Leu Leu Thr Leu Pro Val
Ala Leu Pro
            260                     265
270

Val Gly Gln Leu Leu Glu Leu Ala Ala Arg Pro Gly Arg
Leu Arg Glu
        275                     280                     285

Arg Val Leu Glu Leu Ala Arg Gly Gly Gly Asp Pro Tyr
Ser Asp Leu
    290                     295                     300

Ser Lys Gly Val Leu Arg Cys Arg Thr Val Glu Asp Val
Leu Thr Pro
305                     310                     315
        320

Leu Glu Asp Cys Phe Met Leu Asp Ala Ser Thr Val Leu
Asp Phe Gly
                325                     330
    335

Val Leu Ala Ser Ile Met Gln Ser Gly His Thr Arg Ile
Pro Val Tyr
            340                     345
350

Glu Glu Glu Arg Ser Asn Ile Val Asp Met Leu Tyr Leu
Lys Asp Leu
        355                     360                     365

Ala Phe Val Asp Pro Glu Asp Cys Thr Pro Leu Ser Thr
Ile Thr Arg

370                 375                 380

Phe Tyr Asn His Pro Leu His Phe Val Phe Asn Asp Thr
Lys Leu Asp
385                 390                 395
        400

Ala Val Leu Glu Glu Phe Lys Arg Gly Lys Ser His Leu
Ala Ile Val
                405                 410
    415

Gln Lys Val Asn Asn Glu Gly Glu Gly Asp Pro Phe Tyr
Glu Val Leu
            420                 425
430

Gly Leu Val Thr Leu Glu Asp Val Ile Glu Glu Ile Ile
Arg Ser Glu
        435                 440                 445

Ile Leu Asp Glu Ser Glu Asp Tyr Arg Asp Thr Val Val
Lys Arg Lys
    450                 455                 460

Pro Ala Ser Leu Met Ala Pro Leu Lys Arg Lys Glu Glu
Phe Ser Leu
465                 470                 475
        480

Phe Lys Val Ser Asp Asp Glu Tyr Lys Val Thr Ile Ser
Pro Gln Leu
                485                 490
    495

Leu Leu Ala Thr Gln Arg Phe Leu Ser Arg Glu Val Asp
Val Phe Ser
            500                     505
510

Pro Leu Arg Ile Ser Glu Lys Val Leu Leu His Leu Leu
Lys His Pro
        515                     520                     525

Ser Val Asn Gln Glu Val Arg Phe Asp Glu Ser Asn Arg
Leu Ala Thr
    530                     535                     540

His His Tyr Leu Tyr Gln Arg Ser Gln Pro Val Asp Tyr
Phe Ile Leu
545                     550                     555
        560

Ile Leu Gln Gly Arg Val Glu Val Glu Ile Gly Lys Glu
Gly Leu Lys
                565                     570
    575

Phe Glu Asn Gly Ala Phe Thr Tyr Tyr Gly Val Ser Ala
Leu Thr Val
            580                     585
590

Pro Ser Ser Val His Gln Ser Pro Val Ser Ser Leu Gln
Pro Ile Arg
        595                     600                     605

His Asp Leu Gln Pro Asp Pro Gly Asp Gly Thr His Ser
Ser Ala Tyr
    610                     615                     620

Cys Pro Asp Tyr Thr Val Arg Ala Leu Ser Asp Leu Gln
Leu Ile Lys
625                 630                 635
        640

Val Thr Arg Leu Gln Tyr Leu Asn Ala Leu Leu Ala Thr
Arg Ala Gln
                645                 650
    655

Asn Leu Pro Gln Ser Pro Glu Asn Thr Asp Leu Gln Val
Ile Pro Gly
            660                 665
670

Ser Gln Thr Arg Leu Leu Gly Glu Lys Thr Thr Thr Ala
Ala Gly Ser
        675                 680                 685

Ser His Ser Arg Pro Gly Val Pro Val Glu Gly Ser Pro
Gly Arg Asn
    690                 695                 700

Pro Gly Val
705

<210>  27
<211>  501
<212>  PRT
<213>  Homo sapiens

<400>  27

Met Val Arg Lys Pro Val Val Ser Thr Ile Ser Lys Gly
Gly Tyr Leu
1               5                   10

15

Gln Gly Asn Val Asn Gly Arg Leu Pro Ser Leu Gly Asn
Lys Glu Pro
            20                  25
30

Pro Gly Gln Glu Lys Val Gln Leu Lys Arg Lys Val Thr
Leu Leu Arg
        35                  40                  45

Gly Val Ser Ile Ile Ile Gly Thr Ile Ile Gly Ala Gly
Ile Phe Ile
    50                  55                  60

Ser Pro Lys Gly Val Leu Gln Asn Thr Gly Ser Val Gly
Met Ser Leu
65                  70                  75
        80

Thr Ile Trp Thr Val Cys Gly Val Leu Ser Leu Phe Gly
Ala Leu Ser
                85                  90
    95

Tyr Ala Glu Leu Gly Thr Thr Ile Lys Lys Ser Gly Gly
His Tyr Thr
            100                 105
110

Tyr Ile Leu Glu Val Phe Gly Pro Leu Pro Ala Phe Val
Arg Val Trp
        115                 120                 125

Val Glu Leu Leu Ile Ile Arg Pro Ala Ala Thr Ala Val

Ile Ser Leu
    130                 135                 140

Ala Phe Gly Arg Tyr Ile Leu Glu Pro Phe Phe Ile Gln
Cys Glu Ile
145                 150                 155
        160

Pro Glu Leu Ala Ile Lys Leu Ile Thr Ala Val Gly Ile
Thr Val Val
                165                 170
    175

Met Val Leu Asn Ser Met Ser Val Ser Trp Ser Ala Arg
Ile Gln Ile
            180                 185
190

Phe Leu Thr Phe Cys Lys Leu Thr Ala Ile Leu Ile Ile
Ile Val Pro
        195                 200                 205

Gly Val Met Gln Leu Ile Lys Gly Gln Thr Gln Asn Phe
Lys Asp Ala
    210                 215                 220

Phe Ser Gly Arg Asp Ser Ser Ile Thr Arg Leu Pro Leu
Ala Phe Tyr
225                 230                 235
        240

Tyr Gly Met Tyr Ala Tyr Ala Gly Trp Phe Tyr Leu Asn
Phe Val Thr
                245                 250
    255

Glu Glu Val Glu Asn Pro Glu Lys Thr Ile Pro Leu Ala
Ile Cys Ile
            260                 265
270

Ser Met Ala Ile Val Thr Ile Gly Tyr Val Leu Thr Asn
Val Ala Tyr
        275                 280                 285

Phe Thr Thr Ile Asn Ala Glu Glu Leu Leu Leu Ser Asn
Ala Val Ala
    290                 295                 300

Val Thr Phe Ser Glu Arg Leu Leu Gly Asn Phe Ser Leu
Ala Val Pro
305                 310                 315
        320

Ile Phe Val Ala Leu Ser Cys Phe Gly Ser Met Asn Gly
Gly Val Phe
                325                 330
    335

Ala Val Ser Arg Leu Phe Tyr Val Ala Ser Arg Glu Gly
His Leu Pro
            340                 345
350

Glu Ile Leu Ser Met Ile His Val Arg Lys His Thr Pro
Leu Pro Ala
        355                 360                 365

Val Ile Val Leu His Pro Leu Thr Met Ile Met Leu Phe
Ser Gly Asp
    370                 375                 380

Leu Asp Ser Leu Leu Asn Phe Leu Ser Phe Ala Arg Trp
Leu Phe Ile
385                 390                 395
        400

Gly Leu Ala Val Ala Gly Leu Ile Tyr Leu Arg Tyr Lys
Cys Pro Asp
                405                 410
    415

Met His Arg Pro Phe Lys Val Pro Leu Phe Ile Pro Ala
Leu Phe Ser
            420                 425
430

Phe Thr Cys Leu Phe Met Val Ala Leu Ser Leu Tyr Ser
Asp Pro Phe
        435                 440                 445

Ser Thr Gly Ile Gly Phe Val Ile Thr Leu Thr Gly Val
Pro Ala Tyr
    450                 455                 460

Tyr Leu Phe Ile Ile Trp Asp Lys Lys Pro Arg Trp Phe
Arg Ile Met
465                 470                 475
        480

Ser Glu Lys Ile Thr Arg Thr Leu Gln Ile Ile Leu Glu
Val Val Pro
                485                 490
    495

Glu Glu Asp Lys Leu

            500

<210>  28
<211>  905
<212>  PRT
<213>  Homo sapiens

<400>  28

<210>  29
<211>  474
<212>  PRT
<213>  Homo sapiens

<400>  29

Met Glu Leu Pro Ser Gly Pro Gly Pro Glu Arg Leu Phe
Asp Ser His
1               5                   10
    15

Arg Leu Pro Gly Asp Cys Phe Leu Leu Leu Val Leu Leu
Leu Tyr Ala
            20                  25
30

Pro Val Gly Phe Cys Leu Leu Val Leu Arg Leu Phe Leu
Gly Ile His
        35                  40                  45

Val Phe Leu Val Ser Cys Ala Leu Pro Asp Ser Val Leu
Arg Arg Phe
    50                  55                  60

Val Val Arg Thr Met Cys Ala Val Leu Gly Leu Val Ala
Arg Gln Glu
65                  70                  75
        80

Asp Ser Gly Leu Arg Asp His Ser Val Arg Val Leu Ile
Ser Asn His
                85                  90
    95

Val Thr Pro Phe Asp His Asn Ile Val Asn Leu Leu Thr
Thr Cys Ser
            100                 105
110

Thr Val Ser Glu Ser Glu Ala Glu Ser Ala Thr Gly Arg
Phe Pro Gly
        115                 120                 125

Ala Gln Leu Lys Ala Pro Leu Ser Pro Leu Ala Phe Pro
Met Glu Asp
    130                 135                 140

Thr Glu Leu Pro Leu Thr Pro Ile Leu Tyr Pro Thr Cys
Gln Phe Phe
145                 150                 155

160

Phe Ile Phe Leu Asn Ile Phe Leu Leu Ala Phe Ser Ser
Pro Gly Ser
                165                 170
    175

Gln Pro Leu Leu Asn Ser Pro Pro Ser Phe Val Cys Trp
Ser Arg Gly
            180                 185
190

Phe Met Glu Met Asn Gly Arg Gly Glu Leu Val Glu Ser
Leu Lys Arg
        195                 200                 205

Phe Cys Ala Ser Thr Arg Leu Pro Pro Thr Pro Leu Leu
Leu Phe Pro
    210                 215                 220

Glu Glu Glu Ala Thr Asn Gly Arg Glu Gly Leu Leu Arg
Phe Ser Ser
225                 230                 235
        240

Trp Pro Phe Ser Ile Gln Asp Val Val Gln Pro Leu Thr
Leu Gln Val
                245                 250
    255

Gln Arg Pro Leu Val Ser Val Thr Val Ser Asp Ala Ser
Trp Val Ser
            260                 265
270

Glu Leu Leu Trp Ser Leu Phe Val Pro Phe Thr Val Tyr

Gln Val Arg
        275                 280                 285

Trp Leu Arg Pro Val His Arg Gln Leu Gly Glu Ala Asn
Glu Glu Phe
    290                 295                 300

Ala Leu Arg Val Gln Gln Leu Val Ala Lys Glu Leu Gly
Gln Thr Gly
305                 310                 315
        320

Thr Arg Leu Thr Pro Ala Asp Lys Ala Glu His Met Lys
Arg Gln Arg
                325                 330
    335

His Pro Arg Leu Arg Pro Gln Ser Ala Gln Ser Ser Phe
Pro Pro Ser
            340                 345
350

Pro Gly Pro Ser Pro Asp Val Gln Leu Ala Thr Leu Ala
Gln Arg Val
        355                 360                 365

Lys Glu Val Leu Pro His Val Pro Leu Gly Val Ile Gln
Arg Asp Leu
    370                 375                 380

Ala Lys Thr Gly Cys Val Asp Leu Thr Ile Thr Asn Leu
Leu Glu Gly
385                 390                 395
        400

Ala Val Ala Phe Met Pro Glu Asp Ile Thr Lys Gly Thr
Gln Ser Leu
                405                 410
    415

Pro Thr Ala Ser Ala Ser Lys Phe Pro Ser Ser Gly Pro
Val Thr Pro
            420                 425
430

Gln Pro Thr Ala Leu Thr Phe Ala Lys Ser Ser Trp Ala
Arg Gln Glu
        435                 440                 445

Ser Leu Gln Glu Arg Lys Gln Ala Leu Tyr Glu Tyr Ala
Arg Arg Arg
    450                 455                 460

Phe Thr Glu Arg Arg Ala Gln Glu Ala Asp
465                 470

<210>  30
<211>  532
<212>  PRT
<213>  Homo sapiens

<400>  30

<210>  31
<211>  910
<212>  PRT
<213>  Homo sapiens

<400>  31

Met Lys Lys Met Ser Arg Asn Val Leu Leu Gln Met Glu
Glu Glu Glu
1               5                   10
    15

Asp Asp Asp Asp Gly Asp Ile Val Leu Glu Asn Leu Gly
Gln Thr Ile
            20                  25
30

Val Pro Asp Leu Gly Ser Leu Glu Ser Gln His Asp Phe
Arg Thr Pro
        35                  40                  45

Glu Phe Glu Glu Phe Asn Gly Lys Pro Asp Ser Leu Phe
Phe Asn Asp
    50                  55                  60

Gly Gln Arg Arg Ile Asp Phe Val Leu Val Tyr Glu Asp
Glu Ser Arg
65                  70                  75
        80

Lys Glu Thr Asn Lys Lys Gly Thr Asn Glu Lys Gln Arg

Arg Lys Arg
                85                  90
    95

Gln Ala Tyr Glu Ser Asn Leu Ile Cys His Gly Leu Gln
Leu Glu Ala
            100                 105
110

Thr Arg Ser Val Leu Asp Asp Lys Leu Val Phe Val Lys
Val His Ala
        115                 120                 125

Pro Trp Glu Val Leu Cys Thr Tyr Ala Glu Ile Met His
Ile Lys Leu
    130                 135                 140

Pro Leu Lys Pro Asn Asp Leu Lys Asn Arg Ser Ser Ala
Phe Gly Thr
145                 150                 155
        160

Leu Asn Trp Phe Thr Lys Val Leu Ser Val Asp Glu Ser
Ile Ile Lys
                165                 170
    175

Pro Glu Gln Glu Phe Phe Thr Ala Pro Phe Glu Lys Asn
Arg Met Asn
            180                 185
190

Asp Phe Tyr Ile Val Asp Arg Asp Ala Phe Phe Asn Pro
Ala Thr Arg
        195                 200                 205

Ser Arg Ile Val Tyr Phe Ile Leu Ser Arg Val Lys Tyr
Gln Val Ile
    210                 215                 220

Asn Asn Val Ser Lys Phe Gly Ile Asn Arg Leu Val Asn
Ser Gly Ile
225                 230                 235
        240

Tyr Lys Ala Ala Phe Pro Leu His Asp Cys Lys Phe Arg
Arg Gln Ser
                245                 250
    255

Glu Asp Pro Ser Cys Pro Asn Glu Arg Tyr Leu Leu Tyr
Arg Glu Trp
            260                 265
270

Ala His Pro Arg Ser Ile Tyr Lys Lys Gln Pro Leu Asp
Leu Ile Arg
        275                 280                 285

Lys Tyr Tyr Gly Glu Lys Ile Gly Ile Tyr Phe Ala Trp
Leu Gly Tyr
    290                 295                 300

Tyr Thr Gln Met Leu Leu Leu Ala Ala Val Val Gly Val
Ala Cys Phe
305                 310                 315
        320

Leu Tyr Gly Tyr Leu Asn Gln Asp Asn Cys Thr Trp Ser
Lys Glu Val
                325                 330

335

Cys His Pro Asp Ile Gly Gly Lys Ile Ile Met Cys Pro
Gln Cys Asp
340 345
350

Arg Leu Cys Pro Phe Trp Lys Leu Asn Ile Thr Cys Glu
Ser Ser Lys
355 360 365

Lys Leu Cys Ile Phe Asp Ser Phe Gly Thr Leu Val Phe
Ala Val Phe
370 375 380

Met Gly Val Trp Val Thr Leu Phe Leu Glu Phe Trp Lys
Arg Arg Gln
385 390 395
400

Ala Glu Leu Glu Tyr Glu Trp Asp Thr Val Glu Leu Gln
Gln Glu Glu
405 410
415

Gln Ala Arg Pro Glu Tyr Glu Ala Arg Cys Thr His Val
Val Ile Asn
420 425
430

Glu Ile Thr Gln Glu Glu Glu Arg Ile Pro Phe Thr Ala
Trp Gly Lys
435 440 445

Cys Ile Arg Ile Thr Leu Cys Ala Ser Ala Val Phe Phe

Trp Ile Leu
    450                 455                 460

Leu Ile Ile Ala Ser Val Ile Gly Ile Ile Val Tyr Arg
Leu Ser Val
465                 470                 475
        480

Phe Ile Val Phe Ser Ala Lys Leu Pro Lys Asn Ile Asn
Gly Thr Asp
                485                 490
    495

Pro Ile Gln Lys Tyr Leu Thr Pro Gln Thr Ala Thr Ser
Ile Thr Ala
            500                 505
510

Ser Ile Ile Ser Phe Ile Ile Ile Met Ile Leu Asn Thr
Ile Tyr Glu
        515                 520                 525

Lys Val Ala Ile Met Ile Thr Asn Phe Glu Leu Pro Arg
Thr Gln Thr
    530                 535                 540

Asp Tyr Glu Asn Ser Leu Thr Met Lys Met Phe Leu Phe
Gln Phe Val
545                 550                 555
        560

Asn Tyr Tyr Ser Ser Cys Phe Tyr Ile Ala Phe Phe Lys
Gly Lys Phe
                565                 570
    575

Val Gly Tyr Pro Gly Asp Pro Val Tyr Trp Leu Gly Lys
Tyr Arg Asn
580 585
590

Glu Glu Cys Asp Pro Gly Gly Cys Leu Leu Glu Leu Thr
Thr Gln Leu
595 600 605

Thr Ile Ile Met Gly Gly Lys Ala Ile Trp Asn Asn Ile
Gln Glu Val
610 615 620

Leu Leu Pro Trp Ile Met Asn Leu Ile Gly Arg Phe His
Arg Val Ser
625 630 635
640

Gly Ser Glu Lys Ile Thr Pro Arg Trp Glu Gln Asp Tyr
His Leu Gln
645 650
655

Pro Met Gly Lys Leu Gly Leu Phe Tyr Glu Tyr Leu Glu
Met Ile Ile
660 665
670

Gln Phe Gly Phe Val Thr Leu Phe Val Ala Ser Phe Pro
Leu Ala Pro
675 680 685

Leu Leu Ala Leu Val Asn Asn Ile Leu Glu Ile Arg Val
Asp Ala Trp
690 695 700

Lys Leu Thr Thr Gln Phe Arg Arg Leu Val Pro Glu Lys
Ala Gln Asp
705                 710                 715
        720

Ile Gly Ala Trp Gln Pro Ile Met Gln Gly Ile Ala Ile
Leu Ala Val
                725                 730
    735

Val Thr Asn Ala Met Ile Ile Ala Phe Thr Ser Asp Met
Ile Pro Arg
            740                 745
750

Leu Val Tyr Tyr Trp Ser Phe Ser Val Pro Pro Tyr Gly
Asp His Thr
        755                 760                 765

Ser Tyr Thr Met Glu Gly Tyr Ile Asn Asn Thr Leu Ser
Ile Phe Lys
    770                 775                 780

Val Ala Asp Phe Lys Asn Lys Ser Lys Gly Asn Pro Tyr
Ser Asp Leu
785                 790                 795
        800

Gly Asn His Thr Thr Cys Arg Tyr Arg Asp Phe Arg Tyr
Pro Pro Gly
                805                 810
    815

His Pro Gln Glu Tyr Lys His Asn Ile Tyr Tyr Trp His

Val Ile Ala
            820                 825
830

Ala Lys Leu Ala Phe Ile Ile Val Met Glu His Val Ile
Tyr Ser Val
        835                 840                 845

Lys Phe Phe Ile Ser Tyr Ala Ile Pro Asp Val Ser Lys
Arg Thr Lys
    850                 855                 860

Ser Lys Ile Gln Arg Glu Lys Tyr Leu Thr Gln Lys Leu
Leu His Glu
865                 870                 875
        880

Asn His Leu Lys Asp Met Thr Lys Asn Met Gly Val Ile
Ala Glu Arg
                885                 890
    895

Met Ile Glu Ala Val Asp Asn Asn Leu Arg Pro Lys Ser
Glu
            900                 905
910

<210>  32
<211>  879
<212>  PRT
<213>  Homo sapiens

<400>  32

Met Gly Arg Leu Ala Ser Arg Pro Leu Leu Leu Ala Leu
Leu Ser Leu
1               5                   10
    15

Ala Leu Cys Arg Gly Arg Val Val Arg Val Pro Thr Ala
Thr Leu Val
            20                  25
30

Arg Val Val Gly Thr Glu Leu Val Ile Pro Cys Asn Val
Ser Asp Tyr
        35                  40                  45

Asp Gly Pro Ser Glu Gln Asn Phe Asp Trp Ser Phe Ser
Ser Leu Gly
    50                  55                  60

Ser Ser Phe Val Glu Leu Ala Ser Thr Trp Glu Val Gly
Phe Pro Ala
65                  70                  75
        80

Gln Leu Tyr Gln Glu Arg Leu Gln Arg Gly Glu Ile Leu
Leu Arg Arg
                85                  90
    95

Thr Ala Asn Asp Ala Val Glu Leu His Ile Lys Asn Val
Gln Pro Ser
            100                 105
110

Asp Gln Gly His Tyr Lys Cys Ser Thr Pro Ser Thr Asp
Ala Thr Val
        115                 120                 125

Gln Gly Asn Tyr Glu Asp Thr Val Gln Val Lys Val Leu
Ala Asp Ser

130                 135                 140

Leu His Val Gly Pro Ser Ala Arg Pro Pro Pro Ser Leu
Ser Leu Arg
145                 150                 155
        160

Glu Gly Glu Pro Phe Glu Leu Arg Cys Thr Ala Ala Ser
Ala Ser Pro
                165                 170
    175

Leu His Thr His Leu Ala Leu Leu Trp Glu Val His Arg
Gly Pro Ala
            180                 185
190

Arg Arg Ser Val Leu Ala Leu Thr His Glu Gly Arg Phe
His Pro Gly
        195                 200                 205

Leu Gly Tyr Glu Gln Arg Tyr His Ser Gly Asp Val Arg
Leu Asp Thr
    210                 215                 220

Val Gly Ser Asp Ala Tyr Arg Leu Ser Val Ser Arg Ala
Leu Ser Ala
225                 230                 235
        240

Asp Gln Gly Ser Tyr Arg Cys Ile Val Ser Glu Trp Ile
Ala Glu Gln
                245                 250
    255

Gly Asn Trp Gln Glu Ile Gln Glu Lys Ala Val Glu Val
Ala Thr Val
            260                 265
270

Val Ile Gln Pro Ser Val Leu Arg Ala Ala Val Pro Lys
Asn Val Ser
        275                 280                 285

Val Ala Glu Gly Lys Glu Leu Asp Leu Thr Cys Asn Ile
Thr Thr Asp
    290                 295                 300

Arg Ala Asp Asp Val Arg Pro Glu Val Thr Trp Ser Phe
Ser Arg Met
305                 310                 315
        320

Pro Asp Ser Thr Leu Pro Gly Ser Arg Val Leu Ala Arg
Leu Asp Arg
                325                 330
    335

Asp Ser Leu Val His Ser Ser Pro His Val Ala Leu Ser
His Val Asp
            340                 345
350

Ala Arg Ser Tyr His Leu Leu Val Arg Asp Val Ser Lys
Glu Asn Ser
        355                 360                 365

Gly Tyr Tyr Tyr Cys His Val Ser Leu Trp Ala Pro Gly
His Asn Arg
    370                 375                 380

Ser Trp His Lys Val Ala Glu Ala Val Ser Ser Pro Ala
Gly Val Gly
385                 390                 395
        400

Val Thr Trp Leu Glu Pro Asp Tyr Gln Val Tyr Leu Asn
Ala Ser Lys
            405                 410
    415

Val Pro Gly Phe Ala Asp Asp Pro Thr Glu Leu Ala Cys
Arg Val Val
        420                 425
430

Asp Thr Lys Ser Gly Glu Ala Asn Val Arg Phe Thr Val
Ser Trp Tyr
        435                 440                 445

Tyr Arg Met Asn Arg Arg Ser Asp Asn Val Val Thr Ser
Glu Leu Leu
    450                 455                 460

Ala Val Met Asp Gly Asp Trp Thr Leu Lys Tyr Gly Glu
Arg Ser Lys
465                 470                 475
        480

Gln Arg Ala Gln Asp Gly Asp Phe Ile Phe Ser Lys Glu
His Thr Asp
            485                 490
    495

Thr Phe Asn Phe Arg Ile Gln Arg Thr Thr Glu Glu Asp
Arg Gly Asn

500                     505
510

Tyr Tyr Cys Val Val Ser Ala Trp Thr Lys Gln Arg Asn
Asn Ser Trp
        515                     520                     525

Val Lys Ser Lys Asp Val Phe Ser Lys Pro Val Asn Ile
Phe Trp Ala
    530                     535                     540

Leu Glu Asp Ser Val Leu Val Val Lys Ala Arg Gln Pro
Lys Pro Phe
545                     550                     555
        560

Phe Ala Ala Gly Asn Thr Phe Glu Met Thr Cys Lys Val
Ser Ser Lys
                565                     570
    575

Asn Ile Lys Ser Pro Arg Tyr Ser Val Leu Ile Met Ala
Glu Lys Pro
            580                     585
590

Val Gly Asp Leu Ser Ser Pro Asn Glu Thr Lys Tyr Ile
Ile Ser Leu
        595                     600                     605

Asp Gln Asp Ser Val Val Lys Leu Glu Asn Trp Thr Asp
Ala Ser Arg
    610                     615                     620

Val Asp Gly Val Val Leu Glu Lys Val Gln Glu Asp Glu
Phe Arg Tyr
625                 630                 635
        640

Arg Met Tyr Gln Thr Gln Val Ser Asp Ala Gly Leu Tyr
Arg Cys Met
                645                 650
    655

Val Thr Ala Trp Ser Pro Val Arg Gly Ser Leu Trp Arg
Glu Ala Ala
            660                 665
670

Thr Ser Leu Ser Asn Pro Ile Glu Ile Asp Phe Gln Thr
Ser Gly Pro
        675                 680                 685

Ile Phe Asn Ala Ser Val His Ser Asp Thr Pro Ser Val
Ile Arg Gly
    690                 695                 700

Asp Leu Ile Lys Leu Phe Cys Ile Ile Thr Val Glu Gly
Ala Ala Leu
705                 710                 715
        720

Asp Pro Asp Asp Met Ala Phe Asp Val Ser Trp Phe Ala
Val His Ser
                725                 730
    735

Phe Gly Leu Asp Lys Ala Pro Val Leu Leu Ser Ser Leu
Asp Arg Lys
            740                 745
750

Gly Ile Val Thr Thr Ser Arg Arg Asp Trp Lys Ser Asp
Leu Ser Leu
        755                 760                 765

Glu Arg Val Ser Val Leu Glu Phe Leu Leu Gln Val His
Gly Ser Glu
    770                 775                 780

Asp Gln Asp Phe Gly Asn Tyr Tyr Cys Ser Val Thr Pro
Trp Val Lys
785                 790                 795
        800

Ser Pro Thr Gly Ser Trp Gln Lys Glu Ala Glu Ile His
Ser Lys Pro
                805                 810
    815

Val Phe Ile Thr Val Lys Met Asp Val Leu Asn Ala Phe
Lys Tyr Pro
            820                 825
830

Leu Leu Ile Gly Val Gly Leu Ser Thr Val Ile Gly Leu
Leu Ser Cys
        835                 840                 845

Leu Ile Gly Tyr Cys Ser Ser His Trp Cys Cys Lys Lys
Glu Val Gln
    850                 855                 860

Glu Thr Arg Arg Glu Arg Arg Arg Leu Met Ser Met Glu
Met Asp

865 870 875